# EUROPEAN PATENT APPLICATION

(11) **EP 0 962 466 A1**
(43) Date of publication of application: **08.12.1999**
(21) Application number: 99304240.7
(22) Date of filing: 01.06.1999
(51) Int. Cl.: C07K 14/515, C12N 15/00, C07K 16/00, A61K 38/18

(54) **Angiopoietin related gene sequence scarface 3**

(30) Priority: 02.06.1998 US 87716 P; 02.12.1998 US 110620 P; 13.04.1999 US 129065 P; 21.04.1999 US 130381 P
(71) Applicant: ELI LILLY AND COMPANY, Indianapolis, Indiana 46285 (US)
(72) Inventor: Heuer, Josef Georg, Indianapolis, Indiana 46327 (US); Leonard, Rebecca Ann, indianapolis, Indiana 46260 (US); Burgett, Stanley Gene, Indianapolis, Indiana 46227 (US); Rosteck, Paul Robert JR., Indianapolis, Indiana 46237 (US); Sankhavaram, Patanjali Raghavacharya, Carmel, Indiana 46033 (US)
(74) Representative: Denholm, Anna Marie

(57) **Abstract**

The invention provides isolated Scarface 3 nucleic acid compounds, proteins and fragments thereof, said Scarface 3 proteins being related to the angiopoietin family.- Also provided are vectors and transformed heterologous host cells for expressing Scarface 3, methods for identifying compounds that bind and/or modulate the activity of said Scarface 3 proteins, as well as methods for treating cancer and for myelosuppression, and pharmaceutical compositions comprising Scarface 3 or analog thereof.

## Description

### BACKGROUND OF THE INVENTION

This invention relates to recombinant DNA technology. In particular the invention pertains to angiogenesis-related genes, proteins, pharmaceutical compositions, and pharmaceutical and other uses thereof.

Identifying the role of specific proteins in development and differentiation of higher organisms, including humans, has become a major challenge for modern molecular biology. For example, a secreted protein termed "Scabrous" in *Drosophila,* plays an important role in defining positional aspects of developing neurogenic regions. Interestingly, sequence comparisons reveal that Scabrous is related to the angiopoietin family of mammalian proteins, which are necessary for normal angiogenesis, i.e. vascular network formation.

Angiogenesis occurs during embryonic development and continues throughout life. During embryonic development, a primitive vascular network forms by processes that involve regulated proliferation, differentiation, migration, and association of endothelial cells. Subsequent angiogenic processes remodel this primary network to form a mature cardiovascular system.

During adulthood angiogenesis continues to be important, for example, in normal tissue repair and during the menstrual cycle. Unfortunately, the process also facilitates undesirable pathogenic effects, for example, the proliferation and metastasis of tumor cells.

Mechanistically, angiogenesis involves multiple proteins, including tyrosine kinase receptors and protein ligands that bind the receptors to stimulate or inhibit angiogenesis. See e.g. J. Folkman and P. D'Amore, Cell 87, 1153-1155, 1996; S. Davies et al. Cell, 87, 1161-1169, 1996, herein incorporated by reference. The first protein to be identified in this process was dubbed "angiopoietin 1" (See S. Davis et al. Cell, 87, 1161-1169, 1996). Angiopoietin 1 comprises a 70 KD glycosylated protein of 498 amino acid residues in human and mouse. There are at least two recognizable domains on this protein, one a fibrinogen-like region at the carboxyl end, and the other a coiled-coil domain at the amino terminus. Coiled-coil regions typically are involved in the assembly of a protein into multimeric structures. It is thought that the coiled-coil region of angiopoietin 1 may serve to assemble the fibrinogen-like domain into a multimeric structure. The fibrinogen-like domain of angiopoietin 1 and other related molecules is probably the receptor binding portion of this class of molecules (See Valenzuela et al. Proc. Nat. Acad. Sci. 96, 1904-1909, 1999).

Angiopoietin 1 appears to function in angiogenesis by binding the Tie2 receptor and promoting tyrosine phosphorylation thereof. Interestingly, angiopoietin 1 does not stimulate the growth of endothelial cells in vitro, as does VEGF and fibroblast growth factor, for example. Thus, angiopoietin 1 would seem to play a role other than as a mitogen. The evidence to date favors a role in regulating the assembly of non-endothelial vessel wall components, and in controlling the maturation and stabilization of blood vessels (See C. Suri et al. Cell, 87, 1171-1180, 1996).

Angiopoietin 1 has a naturally occurring antagonist, termed angiopoietin 2, which blocks the ability of angiopoietin 1 to bind its receptor, Tie 2. Angiopoietin 2 appears to be expressed only at sites of vascular remodeling in the adult. (Maisonpierre et al. Science, 277, 55-60, 1997) The other receptor known to be required for normal angiogenesis is Tiel *(See e.g.* D. Hanahan, *Science,* 277, 48, 1997). The Tie2 tyrosine kinase enables endothelial cells to recruit stromal cells for the encasement of endothelial tubes. Tiel, on the other hand, may be involved in the control of fluid exchange across capillary boundaries, and in hemodynamic stress resistance.

Recently, two additional angiopoietin-related proteins were identified in mouse and human, termed angiopoietin 3 and angiopoietin 4 (See Valenzuela et al. Proc. Nat. Acad. Sci. 96, 1904-1909, 1999). These molecules also bind the Tie2 receptor, angiopoietin 3 as an antagonist, but angiopoietin 4 as an agonist.

While the full complement of molecular players involved in angiogenesis are yet to be defined, it is probable that additional angiopoietin-like ligands and receptors exist. Accordingly, there is a need to provide other angiopoietin related molecules, as well as methods of making and using thereof. The present invention relates to an angiopoietin-like gene and its encoded protein, as well as to pharmaceutical and other uses thereof, for example, as a myelosuppression agent in the treatment of leukemia, or as an anti-tumor agent and modulator of angiogenesis.

### BRIEF SUMMARY OF THE INVENTION

The present invention provides angiopoietin-related nucleic acid molecules and their encoded proteins. The genes and proteins of the invention are referred to herein as "Scarface 3." A specific Scarface 3 gene sequence is designated herein by SEQ ID NO:1, which originates from a human source. The present invention further provides a method for treating malignant and non-malignant tumors, and/or preventing tumor growth using Scarface 3, or protein, or analog, that is structurally and functionally related to Scarface 3. The invention also provides a method for treating leukemias, and/or to suppress the growth of hematopoietic progenitor cells, and/or to prevent the expansion of such cells during chemotherapy or radiotherapy.

The Scarface 3 proteins and peptides are structurally and functionally related to the angiopoietins, and are useful, for example, as potential pharmaceutical compounds for modulating angiogenesis. Scarface 3 is also useful in screening for compounds that bind said proteins, and/or as an antigen to raise a specific antibody, which could provide a tool to modulate angiogenesis, or a means for identifying Scarface 3 and related proteins in tissues or other biological samples.

In one embodiment, the present invention relates to a method for treating leukemias by administration of a therapeutically effective amount of Scarface 3 or analog thereof.

In another embodiment the present invention relates to a method for suppressing the growth of hematopoietic stem cells prior to and/or during chemotherapy or radiotherapy.

In another embodiment, the present invention relates to a method for suppressing tumor growth or treating a patient with a solid tumor, or treating cancer by administering to a mammal in need thereof an effective amount of Scarface 3.

In another embodiment, the present invention relates to a substantially purified Scarface 3 protein or fragment thereof.

In another embodiment, the present invention relates to a substantially purified protein having an amino acid sequence that is encoded by a nucleic acid sequence that hybridizes to SEQ ID NO:1 under high stringency conditions, said protein having Scarface 3 activity.

In another embodiment, the present invention relates to a substantially purified Scarface 3 protein from a human source wherein said protein has an amino acid sequence designated herein as SEQ ID NO:2.

In another embodiment, the present invention relates to an analog of Scarface 3, or a protein that is structurally and functionally related to Scarface 3, said analog or protein designated herein as SEQ ID NO:3.

In still another embodiment, the present invention relates to a substantially purified protein preferably having an amino acid sequence that is at least 70% identical to SEQ ID NO:2, said protein having Scarface 3 activity in vivo or in vitro.

In another embodiment, the present invention relates to chimeric or fusion proteins comprising all or part of a Scarface 3 protein or analog thereof.

In another embodiment, the present invention relates to isolated nucleic acids encoding a Scarface 3 protein.

In another embodiment, the present invention relates to isolated nucleic acids encoding a protein designated herein as SEQ ID NO:2.

In another embodiment, the present invention relates to an isolated nucleic acid that encodes a Scarface 3 protein wherein said nucleic acid is SEQ ID NO:1.

In still another embodiment the present invention relates to a vector comprising an isolated nucleic acid of the present invention, wherein said isolated nucleic acid compound may be operably-linked to a promoter sequence.

In another embodiment, the present invention relates to a host cell containing a vector of the present invention.

In yet another embodiment, the present invention relates to a pharmaceutical formulation comprising as an active ingredient a therapeutically effective amount of an Scarface 3 peptide or protein, associated with one or more pharmaceutically acceptable carriers, excipients, or diluents thereof.

In a further embodiment, the present invention relates to a pharmaceutical formulation comprising an Scarface 3 agonist or antagonist associated with one or more pharmaceutically acceptable carriers, excipients, or diluents thereof.

In another embodiment, the present invention relates to Scarface 3 or a pharmaceutically acceptable salt thereof, for use as a pharmaceutical.

In another embodiment, the present invention relates to Scarface 3, or a pharmaceutically acceptable salt thereof, for use as a pharmaceutical to treat leukemia or to inhibit tumor growth.

In still another embodiment the present invention relates to a method for identifying an antagonist of an Scarface 3 peptide, wherein said antagonist interferes in the binding of said Scarface 3 peptide to its receptor, comprising the steps of: mixing an Scarface 3 preparation with a test sample, or a control sample; monitoring the binding of said Scarface 3 peptide to its receptor, by any suitable means; and comparing the level of binding of Scarface 3 to its receptor in the test sample to the level of said binding in the control sample.

In still another embodiment the present invention relates to a method for identifying an agonist of an Scarface 3 protein or peptide, wherein said agonist promotes or substitutes for Scarface 3 in the binding of a Scarface 3 receptor, comprising the steps of: mixing an Scarface 3 preparation with a test sample, or a control sample; monitoring the binding of said Scarface 3 peptide to its receptor, by any suitable means; and comparing the level of binding of Scarface 3 to its receptor in the test sample to the level of said binding in the control sample.

In another embodiment, the present invention relates to a method for modulating angiogenesis by administration of an Scarface 3 peptide.

In still another embodiment, the present invention relates to an antibody that selectively binds to an Scarface 3 peptide or protein.

### DETAILED DESCRIPTION OF THE INVENTION

### Definitions

The term "analog" or "functional analog" refers to a related modified form of Scarface 3, in which at least one amino acid substitution, deletion, or addition has been made such that said analog retains substantially the same biological activity as the unmodified form, in vivo and/or in vitro.

The term "angiogenesis" refers generally to the formation and differentiation of blood vessels.

The term "antibody" as used herein describes antibodies, fragments of antibodies (such as, but not limited, to Fab, Fab', Fab₂', and Fv fragments), and chimeric, humanized, veneered, resurfaced, or CDR-grafted antibodies capable of binding antigens of a similar nature as the parent antibody molecule from which they are derived.

The term "chimeric protein" or "fusion protein" refers to a protein that is related to Scarface 3 comprising a hybrid protein or peptide comprising part or all of a Scarface 3 protein or analog thereof linked to a heterologous protein. The heterologous protein can be fused to the N-terminus or to the C-terminus of Scarface 3. A preferred chimeric protein comprises the fibrinogen-like domain of a Scarface 3 protein fused to the constant region of an antibody.

The terms "complementary" or "complementarity" as used herein refer to the capacity of purine and pyrimidine nucleotides to associate through hydrogen bonding to form double stranded nucleic acid molecules. The following base pairs establish complementary relationships: guanine and cytosine; adenine and thymine; and adenine and uracil. As used herein, "complementary" means that the aforementioned relationship applies to substantially all base pairs comprising two single-stranded nucleic acid molecules over the entire length of said molecules. "Partially complementary" may refer to the aforementioned relationship in which one of two single-stranded nucleic acid molecules is shorter in length than the other such that a portion of one of the molecules remains single-stranded, or to a situation in which equal length single-stranded molecules do not form complementary base pair relationships at each position.

The term "conservative substitution" or "conservative amino acid substitution" refers to a replacement of one or more native amino acid residue(s) in a protein, as exemplified by Table 1.

The term "fibrinogen-like domain" refers to a highly conserved region typically found at the carboxy end of a protein molecule, said domain being related structurally and/or by sequence to the homologous region of a fibrinogen protein molecule. For example, the fibrinogen-like domain of Scarface 3 resides between the position approximating residues 270 through 460 of SEQ ID NO:2. The fibrinogen-like domain may be important for the biological activity of Scarface 3 and related molecules.

The term "fragment thereof" refers to a fragment, piece, or sub-region of a nucleic acid or protein molecule whose sequence is disclosed herein, such that the fragment comprises 5 or more amino acids, or 10 or more nucleotides that are contiguous in the parent protein or nucleic acid molecule. "Fragment thereof" may or may not retain biological activity. A fragment of a protein disclosed herein could be used as an antigen to raise a specific antibody against the parent protein molecule, or it could retain biological activity as a fragment of the native protein molecule. With reference to a nucleic acid molecule, "fragment thereof" refers to 10 or more contiguous nucleotides, derived from a parent nucleic acid. The term also encompasses the complementary sequence. For example, if the fragment entails the sequence 5'-AGCTAG-3', then "fragment thereof" would also include the complementary sequence, 3'-TCGATC-5'.

The term "functional fragment" or "functionally equivalent fragment", as used herein, refers to a region, or fragment of a full length protein, or sequence of amino acids that, for example, comprises an active site of a protein or enzyme, or a binding/recognition site, or any other amino acid sequence motif, said fragment relating to biological function. Functional fragments are capable of providing a substantially similar biological activity as a native full length protein, *in vivo* or *in vitro, viz*. the capacity to modulate angiogenesis, or the ability to promote phosphorylation of the Tie2 receptor on, for example, epithelial cells. Functional fragments can be made by biochemical means or by cloning technology, or as the natural products of alternative splicing mechanisms.

The term "structurally and functionally related" is used herein to describe peptides or proteins that are related structurally and functionally to Scarface 3. Said proteins, designated herein by SEQ ID NO:3, generally constitute modifications of SEQ ID NO:2 in which conservative or other suitable amino acid changes are introduced by natural mutation or by recombinant DNA techniques, or by other means, or by deletion of one to ten contiguous amino acid residues of SEQ ID NO:2. Generally, structurally and functionally related proteins are greater than 50% identical with SEQ ID NO:2, alternatively greater than 65% identical, preferably between 70% to 100% identical with SEQ ID NO:2; still more preferably between 85% to 95% identical; more preferably still, at least 90% identical; most preferably at least 95% identical; still most preferably between 98% to 100% identical to SEQ ID NO:2. The overall percent identity between nucleic acid molecules or protein molecules is determined by any suitable comparison algorithm, well known to the skilled artisan. The overall percent identity is determined by the number of residues that are identical between optimally aligned nucleic acids (or proteins), divided by the total length (including gaps if present) of the shortest sequence of the pair or group being compared. Percent identity can also refer to, or be expressed as, a local comparison of regions, motifs, or blocks within molecules. Local identities between molecules may be greater than 80% to 90%, while surrounding regions may be less conserved, say less than 50% identical. Such variability of conservation of sequence along the length of molecules can result in low percent identity overall, but a high percent identity within locally defined regions. Examples of locally conserved regions might include binding sites, or catalytic sites. For example, the invention is intended to include analogs of Scarface 3 in which at least 40% identity is preserved with the region comprising residues from about 270 through 460 of SEQ ID NO:2.

Nucleic acid or protein sequences are optimally aligned so as to achieve the greatest degree of similarity, allowing for gaps, using any suitable algorithm, for example, a dynamic programming algorithm (See e.g. Smith and Waterman, J. Mol. Biol. 147, 195 (1985), BLASTA (Altschul et al. J. Mol. Biol. 215, 403 (1990); or FASTA (Lipman & Pearson, Science, 227, 1435 (1985), herein incorporated by reference. Such alignments are carried out with the appropriate algorithmic parameters set to maximize the alignment score obtained for a pair of sequences being compared. For example, using a BLAST algorithm, the Hsp parameter can be set at from 10 to 11 for comparing nucleic acids and at 3 when comparing amino acid sequences. Other parameters that can be varied include the expectation value parameter, most typically set to 10. The skilled artisan is aware of the values most appropriate so as to maximize the alignment between any two or more sequences. Said structurally and functionally related proteins retain the biological activity of Scarface 3, such as the ability to modulate angiogenesis, or the ability to stimulate phosphorylation of Tie2, or to inhibit solid tumor growth in vivo, or the ability to suppress the growth of hematopoietic progenitor cells (*See e.g.* D. Dumont et al. *Oncogene* 7, 1471-1480, 1992).

The term "host cell" refers to any eucaryotic or procaryotic cell that is suitable for propagating and/or expressing a cloned gene contained on a vector that is introduced into said host cell by, for example, transformation or transfection, or the like.

The term "homolog" or "homologous" describes the relationship between different nucleic acid molecules, or amino acid sequences, in which said sequences or molecules are related by partial identity or chemical/physical similarity at one or more blocks or regions within said molecules or sequences.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of homology, the stringency of hybridization, and the length of hybridizing strands.

The term "isolated nucleic acid compound" refers to any RNA or DNA sequence, however constructed or synthesized, which is locationally distinct from its natural location and which comprises a subgenomic and/or subchromosomal fragment

The symbol "N" in a nucleic acid sequence refers to adenine ("A"), guanine ("G"), cytosine ("C"), thymine (T"), or uracil ("U") .

The term "modulate" or "modulating" refers to stimulating or inhibiting the object thereof, for example, stimulating or inhibiting angiogenesis.

The term "nucleic acid probe" or "probe" as used herein is a labeled nucleic acid compound which hybridizes with another nucleic acid compound. "Nucleic acid probe" means a single stranded nucleic acid sequence that will combine with a complementary or partially complementary single stranded target nucleic acid sequence to form a double-stranded molecule. A nucleic acid probe may be an oligonucleotide or a nucleotide polymer. A probe will usually contain a detectable moiety which may be attached to the end(s) of the probe or be internal to the sequence of the probe.

The term "orthologue" or "orthologous" refers to two or more genes or proteins from different organisms that exhibit sequence homology.

The term "paralogue" or "paralogous" refers to two or more genes or proteins within a single organism that exhibit sequence homology.

The term "plasmid" refers to an extrachromosomal genetic element. The plasmids disclosed herein are commercially available, publicly available on an unrestricted basis, or can be constructed from readily available plasmids in accordance with published procedures.

A "primer" is a nucleic acid fragment which functions as an initiating substrate for enzymatic or synthetic elongation of, for example, a nucleic acid molecule.

The term "promoter" refers to a nucleic acid sequence that directs transcription, for example, of DNA to RNA. An inducible promoter is one that is regulatable by environmental signals, such as carbon source, heat, or metal ions, for example. A constitutive promoter generally operates at a constant level and is not regulatable.

The term "recombinant DNA cloning vector" as used herein refers to any autonomously replicating agent, including, but not limited to, plasmids and phages, comprising a DNA molecule to which one or more additional DNA segments can or have been incorporated.

The term "recombinant DNA expression vector" or "expression vector" as used herein refers to any recombinant DNA cloning vector, for example a plasmid or phage, in which a promoter and other regulatory elements are present thereby enabling transcription of an inserted DNA, which may encode a protein.

The term "Scarface 3 activity" or "Scarface 3-like activity" refers to one or more biological activities exhibited by Scarface 3, for example, the ability to inhibit tumor growth in vivo, or tumor cell proliferation in vitro, or the ability to stimulate phosphorylation of Tie 2, or exhibiting a myelosuppressive effect in vivo or in vitro, or any other effect of Scarface 3.

The term "stem cell" refers generally to an unspecialized cell type that gives rise to differentiated cells. For example, hematopoietic stem cells in bone marrow give rise to cells of the blood, e.g. B-cells and red blood cells.

The term "stringency" refers to hybridization conditions. High stringency conditions disfavor non-homologous base pairing. Low stringency conditions have the opposite effect. Stringency may be altered, for example, by temperature and salt concentration.

The term "low stringency" refers to conditions that comprise, for example, a temperature of about 37° C or less, a formamide concentration of less than about 50%, and a moderate to low salt (SSC) concentration; or, alternatively, a temperature of about 50° C or less, and a moderate to high salt (SSPE) concentration, for example 1M NaCl.

The term "high stringency" refers to conditions that comprise, for example, a temperature of about 42° C or less, a formamide concentration of less than about 20%, and a low salt (SSC) concentration; or, alternatively, a temperature of about 65° C, or less, and a low salt (SSPE) concentration. For example, high stringency conditions comprise hybridization in 0.5 M NaHPO₄, 7% sodium dodecyl sulfate (SDS), 1 mM EDTA at 65°C (Ausubel, F.M. et *al.* Current Protocols in Molecular Biology, Vol. I, 1989; Green Inc. New York, at 2.10.3).

The term "SSC" comprises a hybridization and wash solution. A stock 20X SSC solution contains 3M sodium chloride, 0.3M sodium citrate, pH 7.0.

The term "SSPE" comprises a hybridization and wash solution. A 1X SSPE solution contains 180 mM NaCl, 9mM Na₂HPO₄, 0.9 mM NaH₂PO₄ and 1 mM EDTA, pH 7.4.

The term "substantially pure," used in reference to a peptide or protein, means substantial separation from other cellular and non-cellular molecules, including other protein molecules. A substantially pure preparation would be about at least 85% pure; preferably about at least 90% to 95% pure. A "substantially pure" protein can be prepared by a variety of techniques, well known to the skilled artisan, including, for example, the IMAC protein purification method.

The term "vector" as used herein refers to a nucleic acid compound used for introducing exogenous or endogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

### Figure Legend

Figure 1. In vivo tumor suppression by Scarface 3.
   A. EMT6 tumor cell expressing a VEGF construct proliferate in vivo.
   B. EMT6 cells expressing a pcDNA vector proliferate in vivo.
   C. Growth of EMT6 cells expressing a Scarface 3 construct is suppressed in vivo.
   D. EMT6 cells expressing an angiopoietin 2 construct proliferate in vivo.

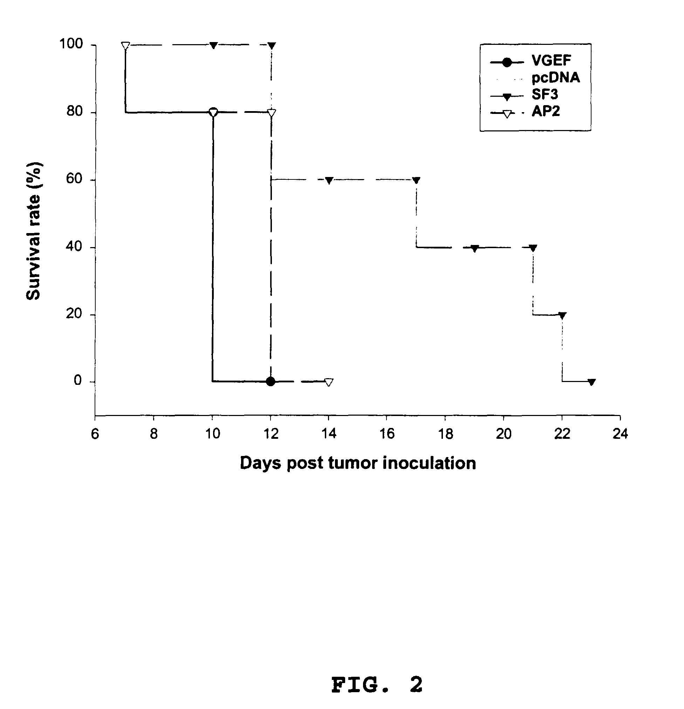

Figure 2. In vivo survival of Balb C mice innoculated with breast tumor cells transfected with Scarface 3.
Figure 3. CFU assay for Scarface 3 transduced BM cells: total colonies.
Figure 4. CFU assay for Scarface 3 transduced BM cells: CFU-E colonies.
Figure 5. CFU assay for Scarface 3 transduced BM cells: CFU-GEMM colonies.
Figure 6. CFU assay for Scarface 3 transduced BM cells: CFU-GM colonies.
Figure 7. Scarface 3 suppresses BM progenitor cell expansion.
Figure 8. Scarface 3 suppresses TF-1 cell proliferation. TF-1 cells were transduced with an IB retrovirus containing a Scarface 3 sequence, or with retrovirus vector IB alone. Cells were selected in blasticidin for 5 days. Approximately 5 x 10⁴ cells were seeded per well of a 96 well tray in the absence or presence of rhGM-CSF. Cells were counted by trypan blue exclusion 48 hours later.
Figure 9. Scarface 3 suppresses TF-1 cell proliferation. TF-1 cells were transduced with an IB retrovirus containing a Scarface 3 sequence, or with retrovirus vector IB alone. Cells were selected in blasticidin for 5 days. Approximately 5 x 10⁴ cells were seeded per well of a 96 well tray in the absence or presence of rhGM-CSF. Cells were pulsed 24 hours later with 1 µCi of ³H-thymidine and harvested for scintillation counting 18 hours later.

The various restriction enzymes disclosed and described herein are commercially available and the manner of use of said enzymes including reaction conditions, cofactors, and other requirements for activity are well known to one of ordinary skill in the art. Reaction conditions for particular enzymes were carried out according to the manufacturer's recommendation.

The Scarface 3 proteins are related by sequence to mammalian angiopoietin 1 and angiopoietin 2, and to the *Drosophila* "Scabrous" protein. Scarface 3 is expressed in multiple tissues and cell types including kidney tubule cells and liver. Those skilled in the art will recognize that owing to the degeneracy of the genetic code, numerous "silent" substitutions of nucleotide base pairs could be introduced into the sequence(s) identified herein without altering the identity of the encoded amino acid(s) or protein or peptide product. All such substitutions are intended to be within the scope of the invention.

Specific examples of a Scarface 3 cDNA and protein are identified herein as SEQ ID NO:1 and SEQ ID NO:2, respectively. Scarface 3 (SEQ ID NO:2) comprises multiple functional domains including a fibrinogen-like domain from approximately about residue 270 through 460, and a coiled-coil domain at the amino terminal end from approximately residues 1 through 269.

Also contemplated by the present invention are analogs of Scarface 3, or proteins and/or peptides that are structurally and functionally related to Scarface 3, identified herein as SEQ ID NO:3. For example, proteins and peptides that are related to SEQ ID NO:2 comprise conservative amino acid substitutions, replacements, deletions, or insertions, at one or more amino acid positions within SEQ ID NO:2, generally in accordance with Table 1. Modifications of Scarface 3 made in accordance with Table 1 are generally expected to retain the biological activity of SEQ ID NO:2 based on the generally recognized substitutability of certain amino acids *(See e.g.* M. Dayhoff, In Atlas of Protein Sequence and Structure, Vol. 5, Supp. 3, pgs 345-352, 1978). Functionality can be tested in an assay that measures phosphorylation of the Tie2 receptor, or the ability to modulate angiogenesis, or to inhibit tumor growth, for example.

**Table 1.**

| ORIGINAL RESIDUE | EXEMPLARY SUBSTITUTIONS |
|---|---|
| ALA | SER, THR |
| ARG | LYS |
| ASN | HIS, SER |
| ASP | GLU, ASN |
| CYS | SER |
| GLN | ASN, HIS |
| GLU | ASP, GLU |
| GLY | ALA, SER |
| HIS | ASN, GLN |
| ILE | LEU, VAL, THR |
| LEU | ILE, VAL |
| LYS | ARG, GLN, GLU, THR |
| MET | LEU, ILE, VAL |
| PHE | LEU, TYR |
| SER | THR, ALA, ASN |
| THR | SER, ALA |
| TRP | ARG, SER |
| TYR | PHE |
| VAL | ILE, LEU, ALA |
| PRO | ALA |

Analogs of Scarface 3, designated herein by SEQ ID NO:3, comprise single or multiple amino acid substitutions in the region between amino acid residues 243 through 425 of SEQ ID NO:2, wherein:
the residue at position 243 is alternatively Pro, Phe, or Trp;
the residue at position 244 is alternatively Ala, Lys, or Arg;
the residue at position 245 is alternatively Glu or Asp;
the residue at position 247 is alternatively Thr, Ala, Gln, or Leu;
the residue at position 248 is alternatively Thr, Asp, Glu, or Gln;
the residue at position 249 is alternatively Ile, Leu, Ala, or Val;
the residue at position 250 is alternatively Tyr, Phe, Leu, or Lys;
the residue at position 251 is alternatively Asn, Gln, Lys, or Glu;
the residue at position 252 is alternatively Arg, Ala, Val, Ser, or Asp;
the residue at position 254 is alternatively Glu, His, or Phe;
the residue at position 255 is alternatively His, Arg, Ser, Asn, Thr, or Asp;
the residue at position 256 is alternatively Thr, Lys, Val or Gln;
the residue at position 257 is alternatively Ser, or Asn;
the residue at position 258 is alternatively Gly or Ser;
the residue at position 259 is alternatively Met, Ile, or Leu;
the residue at position 260 is alternatively Tyr, or Phe;
the residue at position 261 is alternatively Ala, Thr, Leu, Met or Glu;
the residue at position 262 is alternatively Ile, Val or Leu;
the residue at position 263 is alternatively Arg, Lys, Thr, Tyr, or Gln;
the residue at position 264 is alternatively Pro, Phe, or Ile;
the residue at position 265 is alternatively Ser, Gln, Glu or Asn;
the residue at position 266 is alternatively Asn, or Gly;
the residue at position 267 is alternatively Ser, Thr, or Met;
the residue at position 268 is alternatively Gln, Glu, Pro, Arg, or Gly;
the residue at position 269 is alternatively Val, Pro, Leu, or Glu;
the residue at position 270 is alternatively Phe, Met, Ile, or Lys;
the residue at position 271 is alternatively His, Lys, Gln, or Leu;
the residue at position 272 is alternatively Val, Leu, or Ala;
the residue at position 273 is alternatively Tyr, Phe, Trp, or Asn;
the residue at position 275 is alternatively Asp, Asn, Glu or Lys;
the residue at position 276 is alternatively Val, Met, Asn, or Gln;
the residue at position 277 is alternatively Ile, Thr, Ser, Arg, Glu, or Asp;
the residue at position 278 is alternatively Ser, Leu, His, Ala, or Val;
the residue at position 279 is alternatively Gly, Pro, or Asp;
the residue at position 280 is alternatively Ser, or Gly;
the residue at position 281 is alternatively Pro, or Gly;
the residue at position 284 is alternatively Leu, Val or Ile;
the residue at position 287 is alternatively His, Arg, or Lys;
the residue at position 289 is alternatively Ile, Glu, Leu, Thr, or His;
the residue at position 293 is alternatively Gln, Val, or Leu;
the residue at position 294 is alternatively Asn, or Asp;
the residue at position 296 is alternatively Asn, Phe, or Gln;
the residue at position 297 is alternatively Glu, or Arg;
the residue at position 298 is alternatively Thr, Gly, Asn, or Pro;
the residue at position 300 is alternatively Glu, or Lys;
the residue at position 301 is alternatively Asn, Glu, Thr, or Ala;
the residue at position 304 is alternatively Tyr, Met, Val, Gln, Lys, or Ala;
the residue at position 308 is alternatively Arg, Asn, or Asp;
the residue at position 309 is alternatively Leu, Pro, or Ile;
the residue at position 310 is alternatively Asp, Ser, or His;
the residue at position 313 is alternatively Phe, or Tyr;
the residue at position 317 is alternatively Leu, or Asn;
the residue at position 319 is alternatively Lys, Asn, or Phe;
the residue at position 320 is alternatively Ile, or Val;
the residue at position 321 is alternatively Tyr, His, Ser, or Phe;
the residue at position 322 is alternatively Ser, Met, Trp, Gln, or Ala;
the residue at position 323 is alternatively Ile, or Leu;
the residue at position 324 is alternatively Val, Thr, Ser, or Met;
the residue at position 325 is alternatively Lys, Gly, Asn, or Ser;
the residue at position 326 is alternatively Gln, Asp;
the residue at position 327 is alternatively Ser, Arg, Asp, Gly, or Gln;
the residue at position 328 is alternatively Asn, Arg, or Gln;
the residue at position 329 is alternatively Tyr, or Ser;
the residue at position 330 is alternatively Val, Met, Lys, or Arg;
the residue at position 332 is alternatively Arg, Leu, Ala, or Lys;
the residue at position 333 is alternatively Ile, or Val;
the residue at position 334 is alternatively Glu, His, Gln, or Thr;
the residue at position 335 is alternatively Leu, or Met;
the residue at position 336 is alternatively Glu, Arg, Lys, or Met;
the residue at position 339 is alternatively Lys, Ser, Glu, or Asp;
the residue at position 340 is alternatively Asp, or Gly;
the residue at position 341 is alternatively Asn, Lys, or Arg;
the residue at position 342 is alternatively Lys, Ala, Glu, or Arg;
the residue at position 343 is alternatively His, Glu, Val, or Ala;
the residue at position 344 is alternatively Tyr, Phe, or Leu;
the residue at position 345 is alternatively Ile, Leu, Ala, or Ser;
the residue at position 346 is alternatively Glu, Leu, or Gln;
the residue at position 347 is alternatively Tyr, or Phe;
the residue at position 348 is alternatively Ser, Ala, Asp, or Glu;
the residue at position 349 is alternatively Phe, or Val;
the residue at position 350 is alternatively Tyr, His, or Arg;
the residue at position 351 is alternatively Leu, or Ile;
the residue at position 352 is alternatively Gly, Glu, or Ser;
the residue at position 353 is alternatively Asn, Pro, Ser, or Gly;
the residue at position 354 is alternatively His, or Glu;
the residue at position 355 is alternatively Glu, Asp, Ser, or Lys;
the residue at position 356 is alternatively Thr, Gln, Leu, or Glu;
the residue at position 357 is alternatively Asn, Tyr, Phe, or Ala;
the residue at position 359 is alternatively Thr, Ser, Arg, or Lys;
the residue at position 360 is alternatively Leu, or Ile;
the residue at position 361 is alternatively His, Arg, Gln, or Tyr;
the residue at position 363 is alternatively Val, Ala, Gly,or Lys;
the residue at position 364 is alternatively Ala, Gly, Arg, Thr, or Pro;
the residue at position 365 is alternatively Ile, Val, Tyr, Leu, or His;
the residue at position 366 is alternatively Thr, Ala, Gln, or His;
the residue at position 368 is alternatively Asn, Thr, or Gln;
the residue at position 369 is alternatively Val, Ala, or Leu;
the residue at position 370 is alternatively Pro, or Gly;
the residue at position 371 is alternatively Asn, Asp, Ser, or Thr;
the residue at position 372 is alternatively Ala, Ser, or Val;
the residue at position 373 is alternatively Ile, Leu, Phe, Met or Pro;
the residue at position 374 is alternatively Pro, Ile, Ser, Thr, or Met;
the residue at position 375 is alternatively Glu, Trp, Ser, Leu, or Gln;
the residue at position 376 is alternatively Asn, His, Pro, or Gly;
the residue at position 377 is alternatively Lys, Asn, or Leu;
the residue at position 378 is alternatively Asp, Gly, or Ser;
the residue at position 379 is alternatively Leu, Asn, Lys, Ala, or Val;
the residue at position 380 is alternatively Val, Gln, Asp, or Pro;
the residue at position 382 is alternatively Ser, or Thr;
the residue at position 384 is alternatively Trp, Lys, or Leu;
the residue at position 386 is alternatively His, or Asp;
the residue at position 387 is alternatively Lys, Asn, or His;
the residue at position 388 is alternatively Ala, or Asp;
the residue at position 389 is alternatively Lys, Asn, Val, Met, or Leu;
the residue at position 390 is alternatively Gly, Arg, Tyr, or Cys;
the residue at position 391 is alternatively His, Met, Ile, Thr, Ala, or Asp;
the residue at position 392 is alternatively Phe, Cys, Gly, or Lys;
the residue at position 393 is alternatively Asn, or Lys;
the residue at position 395 is alternatively Pro, Ala, or Ser;
the residue at position 396 is alternatively Glu, His, Leu, Gln, or Lys;
the residue at position 397 is alternatively Gly, Ser, Phe, Tyr, or Met;
the residue at position 398 is alternatively Tyr, Leu, Gln, or His;
the residue at position 399 is alternatively Ser, Lys, or Thr;
the residue at position 405 is alternatively His, Phe, or Tyr;
the residue at position 406 is alternatively Asp, Asn, or Gly;
the residue at position 407 is alternatively Glu, Ala, or Thr;
the residue at position 409 is alternatively Gly, Ala, or Ser;
the residue at position 410 is alternatively Glu, His, or pro;
the residue at position 411 is alternatively Asn, or Ser;
the residue at position 416 is alternatively Lys, Gln, Met or Val;
the residue at position 417 is alternatively Tyr, Trp, or Phe;
the residue at position 418 is alternatively Asn, Phe, or Tyr;
the residue at position 419 is alternatively Lys, Arg, Pro, or Thr;
the residue at position 420 is alternatively Pro, Ser, Gly, Gln, or Ala;
the residue at position 421 is alternatively Arg, Ile, Gly, or Arg;
the residue at position 422 is alternatively Ala, Gln, or His;
the residue at position 423 is alternatively Lys, Tyr, Asn, or Gln;
the residue at position 424 is alternatively Ser, Arg, Thr, or His;
the residue at position 425 is alternatively Lys, Gln, Ser, Asn, or Gly;

### Fragments of Scarface 3 proteins

One embodiment of the invention provides fragments of Scarface 3 that may or may not be biologically active. Such fragments are useful, for example, as an antigen for producing an antibody to said proteins.

Fragments of the proteins disclosed herein may be generated by any number of suitable techniques, including chemical synthesis of any portion of SEQ ID NO:2, or SEQ ID NO:3, proteolytic digestion of said proteins, or most preferably, by recombinant DNA mutagenesis techniques, well known to the skilled artisan. *See. e.g.* K. Struhl, "Reverse biochemistry: Methods and applications for synthesizing yeast proteins *in vitro," Meth. Enzymol.* 194, 520-535. In a preferred method, a nested set of deletion mutations are introduced into a gene or cDNA (e.g. SEQ ID NO:1) encoding an Scarface 3 protein such that varying amounts of the peptide coding region are deleted, either from the amino terminal end, or from the carboxyl end of the protein molecule. This method can also be used to create internal fragments of the intact protein in which both the carboxyl and amino terminal ends are removed. Several appropriate nucleases can be used to create such deletions, for example Ba131, or in the case of a single stranded nucleic acid molecule, mung bean nuclease. For simplicity, it is preferred that the Scarface 3 gene be cloned into a single-stranded cloning vector, such as bacteriophage M13, or equivalent. If desired, the resulting deletion fragments can be subcloned into any suitable vector for propagation and expression in any suitable host cell.

Functional fragments of the Scarface 3 proteins may be produced as described above, preferably using cloning techniques to engineer smaller versions of the intact gene or cDNA, lacking sequence from the 5' end, the 3' end, from both ends, or from an internal site. Smaller fragments of the gene or cDNA can be used to produce the encoded peptides, which can be tested for biological activity using any suitable assay, for example, the ability of a protein fragment to stimulate phosphorylation of Tie2, or to inhibit tumor cell growth in vivo or in vitro.

### Gene Isolation Procedures

Those skilled in the art will recognize that the Scarface 3 genes could be obtained by a plurality of recombinant DNA techniques including, for example, hybridization, polymerase chain reaction (PCR) amplification, or *de novo* DNA synthesis. *(See e.g.,* T. Maniatis *et al.* Molecular Cloning: A Laboratory Manual, 2d Ed. Chap. 18 (1989)).

Methods for constructing cDNA libraries in a suitable vector such as a plasmid or phage for propagation in procaryotic or eucaryotic cells are well known to those skilled in the art. [See *e.g.* Maniatis *et al. Supra].* Suitable cloning vectors are well known and are widely available.

The Scarface 3 genes, cDNAs, or fragments thereof, can be isolated from any tissue in which said genes are expressed. For example, Scarface 3 is expressed in kidney and liver tissues.

In one method for gene isolation, mRNA is isolated from a suitable tissue, and first strand cDNA synthesis is carried out. Second round DNA synthesis can be carried out for the production of the second strand. If desired, the double-stranded cDNA can be cloned into any suitable vector, for example, a plasmid, thereby forming a cDNA library. Oligonucleotide primers targeted to any suitable region of the sequences disclosed herein can be used for PCR amplification of Scarface 3 genes. *See e.g.* PCR Protocols: A Guide to Method and Application, Ed. M. Innis et al., Academic Press (1990). The PCR amplification comprises template DNA, suitable enzymes, primers, and buffers, and is conveniently carried out in a DNA Thermal Cycler (Perkin Elmer Cetus, Norwalk, CT). A positive result is determined by an appropriately-sized DNA fragment following agarose gel electrophoresis.

### Protein Production Methods

One embodiment of the present invention relates to substantially purified proteins encoded by Scarface 3 genes or cDNAs. Skilled artisans will recognize that the proteins of the present invention can be synthesized by a number of different methods, such as chemical methods well known in the art, including solid phase peptide synthesis or recombinant methods. Both methods are described in U.S. Patent 4,617,189, incorporated herein by reference.

The principles of solid phase chemical synthesis of polypeptides are well known in the art and may be found in general texts in the area. *See, e.g.*, H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems.

The proteins of the present invention can also be produced by recombinant DNA methods using a cloned Scarface 3 gene as template. Recombinant methods are preferred if a high yield is desired. Expression of an Scarface 3 gene or cDNA can be carried out in a variety of suitable host cells, well known to those skilled in the art. For example, SEQ ID NO:1 is introduced into a host cell by any suitable means, well known to those skilled in the art. While chromosomal integration of the cloned gene is within the scope of the present invention, it is preferred that the gene be cloned into a suitable extra-chromosomally maintained expression vector so that the coding region of the Scarface 3 gene is operably-linked to a constitutive or inducible promoter.

The basic steps in the recombinant production of an Scarface 3 protein (including analog, or fusion protein thereof) are:
a) constructing a natural, synthetic or semi-synthetic DNA encoding an Scarface 3 protein;
b) integrating said DNA into an expression vector in a manner suitable for expressing the Scarface 3 protein, either alone or as a fusion protein;
c) transforming or otherwise introducing said vector into an appropriate eucaryotic or prokaryotic host cell forming a recombinant host cell,
d) culturing said recombinant host cell in a manner to express the Scarface 3 protein; and
e) recovering and substantially purifying the Scarface 3 protein by any suitable means, well known to those skilled in the art.

### Expressing Recombinant Scarface 3 Protein in Procaryotic and Eucaryotic Host Cells

Procaryotes may be employed in the production of recombinant Scarface 3 protein. For example, the *Escherichia coli* K12 strain 294 (ATCC No. 31846) is particularly useful for the prokaryotic expression of foreign proteins. Other strains of *E. coli,* bacilli such as *Bacillus subtilis,* enterobacteriaceae such as *Salmonella typhimurium* or *Serratia marcescens,* various *Pseudomonas* species and other bacteria, such as *Streptomyces,* can also be employed as host cells in the cloning and expression of the recombinant proteins of this invention.

Promoter sequences suitable for driving the expression of genes in procaryotes include β-lactamase [e.g. vector pGX2907, ATCC 39344, contains a replicon and b -lactamase gene], lactose systems [Chang et al., *Nature* (London), 275:615 (1978); Goeddel et al., *Nature* (London), 281:544 (1979)], alkaline phosphatase, and the tryptophan (trp) promoter system [vector pATH1 (ATCC 37695)], which is designed to facilitate expression of an open reading frame as a trpE fusion protein under the control of the trp promoter. Hybrid promoters such as the tac promoter (isolatable from plasmid pDR540, ATCC-37282) are also suitable. Still other bacterial promoters, whose nucleotide sequences are generally known, may be ligated to DNA encoding the protein of the instant invention, using linkers or adapters to supply any required restriction sites. Promoters for use in bacterial systems also will contain a Shine-Dalgarno sequence operably-linked to the DNA encoding the desired polypeptides. These examples are illustrative rather than limiting.

The proteins of this invention may be synthesized either by direct expression or as a fusion protein comprising the Scarface 3 protein of interest as a translational fusion with another protein or peptide which may be removable by enzymatic or chemical cleavage. It is often observed in the production of certain peptides in recombinant systems that expression as a fusion protein prolongs the lifespan, increases the yield of the desired peptide, or provides a convenient means of purifying the protein. This is particularly relevant when expressing mammalian proteins in procaryotic hosts. A variety of peptidases (e.g. enterokinase and thrombin) which cleave a polypeptide at specific sites or digest the peptides from the amino or carboxy termini *(e.g.* diaminopeptidase) of the peptide chain are known. Furthermore, particular chemicals *(e.g.* cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.*, P. Carter, "Site Specific Proteolysis of Fusion Proteins", Chapter 13, in Protein Purification: From Molecular Mechanisms to Large Scale Processes, American Chemical Society, Washington, D.C. (1990).

One useful Scarface 3 fusion protein is a GST fusion protein in which a Scarface 3 protein of the invention is fused to the C-terminus of GST sequences. Such fusion proteins are useful, for example, for purification of a recombinant polypeptide of the invention.

In another embodiment of the invention, the fusion partner comprises an immunoglobulin peptide or protein in which part or all of a Scarface 3 protein or analog, for example, the fibrinogen-like domain, is fused to the constant region of an IgG molecule. Such fusion proteins can facilitate purification and may be useful in pharmaceutical compositions or in screens for compounds that modulate the interaction of Scarface 3 with its receptor.

In addition to procaryotes, a variety of amphibian expression systems such as frog oocytes, and mammalian cell systems can be used. The choice of a particular host cell depends to some extent on the particular expression vector used. Exemplary mammalian host cells suitable for use in the present invention include HepG-2 (ATCC HB 8065), CV-1 (ATCC CCL 70), LC-MK₂ (ATCC CCL 7.1), 3T3 (ATCC CCL 92), CHO-K1 (ATCC CCL 61), HeLa (ATCC CCL 2), RPMI8226 (ATCC CCL 155), H4IIEC3 (ATCC CCL 1600), C127I (ATCC CCL 1616), HS-Sultan (ATCC CCL 1884), and BHK-21 (ATCC CCL 10), for example.

A wide variety of vectors are suitable for transforming mammalian host cells. For example, the pSV2-type vectors comprise segments of the simian virus 40 (SV40) genome required for transcription and polyadenylation. A large number of plasmid pSV2-type vectors have been constructed, such as pSV2-gpt, pSV2-neo, pSV2-dhfr, pSV2-hyg, and pSV2-b-globin, in which the SV40 promoter drives transcription of an inserted gene. These vectors are widely available from sources such as the American Type Culture Collection (ATCC), 12301 Parklawn Drive, Rockville, Maryland, 20852, or the Northern Regional Research Laboratory (NRRL), 1815 N. University Street, Peoria, Illinois, 61604.

Promoters suitable for expression in mammalian cells include the SV40 late promoter, promoters from eukaryotic genes, such as, for example, the estrogen-inducible chicken ovalbumin gene, the interferon genes, the glucocorticoid-inducible tyrosine aminotransferase gene, the thymidine kinase gene promoter, and the promoters of the major early and late adenovirus genes.

Plasmid pRSVcat (ATCC 37152) comprises portions of a long terminal repeat of the Rous Sarcoma virus, a virus known to infect chickens and other host cells. This long terminal repeat contains a promoter which is suitable for use in the vectors of this invention. H. Gorman *et al., Proc. Nat. Acad. Sci. (USA),* 79, 6777 (1982). The plasmid pMSVi (NRRL B-15929) comprises the long terminal repeats of the Murine Sarcoma virus, a virus known to infect mouse and other host cells. The mouse metallothionein promoter has also been well characterized for use in eukaryotic host cells and is suitable for use in the present invention. This promoter is present in the plasmid pdBPV-MMTneo (ATCC 37224) which can serve as the starting material for the construction of other plasmids of the present invention.

Transfection of mammalian cells with vectors can be performed by a plurality of well known processes including, but not limited to, protoplast fusion, calcium phosphate co-precipitation, electroporation and the like. See, e.g., Maniatis *et al., supra.*

Some viruses also make appropriate vectors. Examples include the adenoviruses, the adeno-associated viruses, the vaccinia virus, the herpes viruses, the baculoviruses, and the rous sarcoma virus, as described in U.S. Patent 4,775,624, incorporated herein by reference.

Eucaryotic microorganisms such as yeast and other fungi are also suitable host cells. The yeast *Saccharomyces cerevisiae* is the preferred eucaryotic microorganism. Other yeasts such as *Kluyveromyces lactis* and *Pichia pastoris* are also suitable. For expression in *Saccharomyces,* the plasmid YRp7 (ATCC-40053), for example, may be used. See, e.g., L. Stinchcomb *et al., Nature,* 282, 39 (1979); J. Kingsman *et al., Gene,* 7, 181 (1979); S. Tschemper *et al., Gene,* 10, 157 (1980). Plasmid YRp7 contains the TRP1 gene which provides a selectable marker for use in a trp1 auxotrophic mutant.

### Purification of Recombinantly-Produced Scarface 3 Proteins

An expression vector carrying a cloned Scarface 3 gene or fragment thereof (e.g. SEQ ID NO:1) or a genetic code equivalent thereof is transformed or transfected into a suitable host cell using standard methods. Cells that contain the vector are propagated under conditions suitable for expression of a recombinant Scarface 3 protein. For Example, if the recombinant gene has been placed under the control of an inducible promoter, suitable growth conditions would incorporate the appropriate inducer. The recombinantly-produced protein may be purified from cellular extracts by any suitable means.

In a preferred process for protein purification, an Scarface 3 gene is modified at the 5' end to incorporate several histidine codons. This modification produces an "histidine tag" at the amino terminus of the encoded protein, that enables a single-step protein purification method [i.e. "immobilized metal ion affinity chromatography" (IMAC)], essentially as described in U.S. Patent 4,569,794, which hereby is incorporated by reference. The IMAC method enables rapid isolation of substantially pure recombinant Scarface 3 protein starting from a crude extract of cells that express a modified recombinant protein, as described above.

### Production of Antibodies

The proteins of this invention and fragments thereof may be used in the production of antibodies. The instant invention also encompasses single-chain polypeptide binding molecules.

The production of antibodies, both monoclonal and polyclonal, in animals, especially mice, is well known in the art. See, e.g., C. Milstein, Handbook of Experimental Immunology, (Blackwell Scientific Pub., 1986); J. Goding, Monoclonal Antibodies: Principles and Practice, (Academic Press, 1983). For the production of monoclonal antibodies the basic process begins with injecting a mouse, or other suitable animal, with an immunogen. The mouse is subsequently sacrificed and cells taken from its spleen are fused with myeloma cells, resulting in a hybridoma that reproduces *in vitro.* The population of hybridomas is screened to isolate individual clones, each of which secretes a single antibody species, specific for the immunogen. Each antibody obtained in this way is the clonal product of a single B cell.

Chimeric antibodies are described in U.S. Patent No. 4,816,567, the entire contents of which is herein incorporated by reference. This reference discloses methods and vectors for the preparation of chimeric antibodies. An alternative approach is provided in U.S. Patent No. 4,816,397, the entire contents of which is herein incorporated by reference. This patent teaches coexpression of the heavy and light chains of an antibody in the same host cell.

The approach of U.S. Patent 4,816,397 has been further refined in European Patent Publication No. 0 239 400. The teachings of this European patent publication are a preferred format for genetic engineering of monoclonal antibodies. In this technology the complementarity determining regions (CDRs) of a human antibody are replaced with the CDRs of a murine monoclonal antibody, thereby converting the specificity of the human antibody to the specificity of a murine antibody.

Single chain antibodies and libraries thereof are yet another variety of genetically engineered antibody technology that is well known in the art. *(See, e.g.* R.E. Bird, *et al., Science* 242:423-426 (1988); PCT Publication Nos. WO 88/01649, WO 90/18430, and WO 91/10737. Single chain antibody technology involves covalently joining the binding regions of heavy and light chains to generate a single polypeptide chain. The binding specificity of the intact antibody molecule is thereby reproduced on a single polypeptide chain.

The antibodies contemplated by this invention are useful in diagnostics, therapeutics or in diagnostic/therapeutic combinations.

The proteins of this invention or suitable fragments thereof can be used to generate polyclonal or monoclonal antibodies, and various inter-species hybrids, or humanized antibodies, or antibody fragments, or single-chain antibodies. The techniques for producing antibodies are well known to skilled artisans. (See e.g. A.M. Campbell, Monoclonal Antibody Technology: Laboratory Techniques in Biochemsitry and Molecular Biology, Elsevier Science Publishers, Amsterdam (1984); Kohler and Milstein, *Nature* 256, 495-497 (1975); Monoclonal Antibodies: Principles & Applications Ed. J.R.Birch & E.S. Lennox, Wiley-Liss, 1995.

A protein used as an immunogen may be modified or administered in an adjuvant, by subcutaneous or intraperitoneal injection into, for example, a mouse or a rabbit. For the production of monoclonal antibodies, spleen cells from immunized animals are removed, fused with myeloma cells, such as SP2/0-Ag18 cells, and allowed to become monoclonal antibody producing hybridoma cells in the manner known to the skilled artisan. Hybridomas that secrete a desired antibody molecule can be screened by a variety of well known methods, for example ELISA assay, western blot analysis, or radioimmunoassay (Lutz *et al. Exp. Cell Res.* 175, 109-124 (1988); Monoclonal Antibodies: Principles & Applications Ed. J.R.Birch & E.S. Lennox, Wiley-Liss, 1995).

For some applications labeled antibodies are desirable. Procedures for labeling antibody molecules are widely known, including for example, the use of radioisotopes, affinity labels, such as biotin or avidin, enzymatic labels, for example horseradish peroxidase, and fluorescent labels, such as FITC or rhodamine (See *e.g.* Enzyme-Mediated Immunoassay, Ed. T. Ngo, H. Lenhoff, Plenum Press 1985; Principles of Immunology and Immunodiagnostics, R.M. Aloisi, Lea & Febiger, 1988).

Labeled antibodies are useful for a variety of diagnostic applications. In one embodiment the present invention relates to the use of labeled antibodies to detect the presence of Scarface 3 proteins. Alternatively, the antibodies could be used in a screen to identify potential modulators of Scarface 3. For example, in a competitive displacement assay, the antibody or compound to be tested is labeled by any suitable method. Competitive displacement of an antibody from an antibody-antigen complex by a test compound such that a test compound-antigen complex is formed provides a method for identifying compounds that bind Scarface 3.

Other embodiments of the present invention comprise isolated nucleic acid sequences that encode the proteins of the invention, for example, SEQ ID NO:2 or SEQ ID NO:3. Also contemplated are related nucleic acids that hybridize to a Scarface 3 gene or cDNA under high stringency conditions. For example, the invention includes nucleic acids that hybridize to SEQ ID NO:1 under high stringency conditions. Such sequences may or may not encode functional proteins. In a preferred embodiment, nucleic acids that hybridize to SEQ ID NO:1 under high stringency conditions encode a protein that has similar biological function as Scarface 3, for example the ability to modulate angiogenesis, or the ability to stimulate the phosphorylation of Tie2, or the ability to suppress tumor growth. Such Scarface 3-related sequences may come, for example, from paralogous or orthologous genes.

Hybridization under high stringency conditions can be detected using any suitable method, for example, a filter-based assay such as a Southern blot analysis or a Northern blot analysis, according to well known methods. Specific hybridization means that a signal is detected on a autoradiograph, for example, that is significantly greater than background hybridization and/or to non-related sequences, for example that overall are less than 50% identical with a Scarface 3 probe. Alternatively, hybridization may be detected using a solution-based assay in which the probe and target sequences are in solution during the hybridization reaction. Hybridization means that at least 25% to 50% of the probe molecule remains bound to a target sequence after hybridization, preferably at least 50% remains bound to the target.

A Scarface 3 cDNA of the invention (e.g. SEQ ID NO:1) and related nucleic acid molecules can be produced by chemical synthetic methods. The synthesis of nucleic acids is well known in the art. *See, e.g.,* E.L. Brown, R. Belagaje, M.J. Ryan, and H.G. Khorana, *Methods in Enzymology,* 68:109-151 (1979). Nucleic acids of this invention, including those disclosed herein, could be generated using a conventional DNA synthesizing apparatus, such as the Applied Biosystems Model 380A or 380B DNA synthesizers (Applied Biosystems, Inc., 850 Lincoln Center Drive, Foster City, CA 94404) using phosphoramidite chemistry, thereafter ligating the fragments so as to reconstitute the entire gene. Alternatively, phosphotriester chemistry may be employed to synthesize the nucleic acids of this invention. *(See, e.g.,* M.J. Gait, ed., Oligonucleotide Synthesis, A Practical Approach, (1984)).

In an alternative methodology, namely PCR, the DNA sequences disclosed and described herein, can be produced from a plurality of starting materials. For example, starting with a cDNA preparation (e.g. cDNA library) derived from a tissue that expresses an Scarface 3 gene, suitable oligonucleotide primers complementary to, for example, SEQ ID NO:1, or to a sub-region therein, are prepared as described in U.S. Patent No. 4,889,818, hereby incorporated by reference. Other suitable protocols for the PCR are disclosed in PCR Protocols: A Guide to Method and Applications, Ed. Michael A. Innis et al., Academic Press, Inc. (1990). Using PCR, any region of the Scarface 3 gene(s) can be targeted for amplification such that full or partial length gene sequences may be produced.

The present invention also comprises ribonucleic acids that can be prepared using polynucleotide synthetic methods discussed *supra,* or they may be prepared enzymatically, for example, using RNA polymerase to transcribe a Scarface 3 DNA template, e.g. a nucleic acid comprising SEQ ID NO:1 that will allow for transcription. In one embodiment, a Scarface 3 RNA comprises the RNA version of SEQ ID NO:1 in which thymine residues are substituted by uracil residues.

The most preferred systems for preparing the ribonucleic acids of the present invention use the RNA polymerase from bacteriophage T7 or bacteriophage SP6. These RNA polymerases are highly specific, requiring the insertion of bacteriophage-specific sequences at the 5' end of the template to be transcribed. *See*, Maniatis *et al., supra.*

This invention also provides nucleic acids, RNA or DNA, that are complementary to Scarface 3 genes or fragments thereof, for example, SEQ ID NO:1.

### Nucleic Acid Probes

The present invention also provides probes and primers useful, for example, in hybridization screens of genomic, subgenomic, or cDNA libraries, as well as hybridization against nucleic acids derived from cell lines or tissues. Such hybridization screens are useful as methods to identify and isolate full length, or identical, homologous or functionally related sequences from human or other organisms.

In one embodiment the present invention relates to the use of a nucleic acid disclosed herein or fragment thereof as a probe to identify and isolate other related genes comprising said nucleic acids or related nucleic acids. A nucleic acid compound comprising SEQ ID NO:1, or a complementary sequence thereof, or a fragment thereof, which is at least 18 base pairs in length, and which will selectively hybridize to DNA or mRNA encoding Scarface 3 protein or fragment thereof, or a functionally related protein, is provided. Preferably, the 18 or more base pair compound is DNA. *See e.g.* B. Wallace and G. Miyada, "Oligonucleotide Probes for the Screening of Recombinant DNA Libraries," In *Meth. Enzym.,* 152, 432-442, Academic Press (1987).

Probes and primers can be prepared by enzymatic or recombinant methods, well known to those skilled in the art *(See e.g.* Sambrook et al. *supra).* A probe may be a single stranded nucleic acid sequence which is complementary in some particular degree to a nucleic acid sequence sought to be detected ("target sequence"). A probe may be labeled with a detectable moiety such as a radio-isotope, antigen, or chemiluminescent moiety. A description of the use of nucleic acid hybridization as a procedure for the detection of particular nucleic acid sequences is described in U.S. Patent No. 4,851,330 to Kohne, entitled "Method for Detection, Identification and Quantitation of Non-Viral Organisms."

Having the DNA sequence of the present invention allows preparation of relatively short DNA (or RNA) sequences having the ability to specifically hybridize to nucleic acids that are homologous or identical to sequences disclosed herein. In these aspects, nucleic acid probes of an appropriate length are prepared. The ability of such nucleic acid probes to specifically hybridize to a polynucleotide encoding a Scarface 3 gene or related sequence lends particular utility in a variety of embodiments. Most importantly, the probes may be used in a variety of assays for detecting the presence of complementary sequences in a given sample, in chromosome mapping, in tissue typing, etc.

In certain embodiments, it is advantageous to use oligonucleotide primers. The sequence of such primers is designed using a polynucleotide of the present invention for use in detecting, amplifying or mutating a defined segment of a gene or polynucleotide that encodes an Scarface 3 polypeptide, using PCR technology.

Preferred nucleic acid sequences employed for hybridization studies, or assays, include probe molecules that are complementary to at least an about 18 to an about 70-nucleotide long stretch of a polynucleotide that encedes an Scarface 3 polypeptide. Molecules having complementary sequences over stretches greater than 18 bases in length are generally preferred, in order to increase stability and selectivity of the hybrid. One will generally prefer to design nucleic acid molecules having gene-complementary stretches of 25 to 40 nucleotides, 55 to 70 nucleotides, or even longer where desired. Such fragments may be readily prepared by, for example, directly synthesizing the fragment by chemical means, by application of nucleic acid reproduction technology, such as the PCR TM technology of U.S. Pat. No. 4,603,102, herein incorporated by reference, or by excising selected DNA fragments from recombinant plasmids containing appropriate inserts and suitable restriction enzyme sites.

The following guidelines are useful for designing probes with desirable characteristics. The extent and specificity of hybridization reactions are affected by a number of factors that determine the sensitivity and specificity of a particular probe, whether perfectly complementary to its target or not. The affect of various experimental parameters and conditions are well known to those skilled in the art.

First, the stability of the probe:target nucleic acid hybrid should be chosen to be compatible with the assay conditions. This may be accomplished by avoiding long A and T rich sequences, by terminating the hybrids with G:C base pairs and by designing a probe with an appropriate Tm (i.e. melting temperature). The melting profile, including the Tm of a hybrid comprising an oligonucleotide and target sequence, may be determined using a Hybridization Protection Assay. The probe should be chosen so that the length and % GC content result in a Tm about 2°-10° C higher than the temperature at which the final assay will be performed. The base composition of the probe is also a significant factor because G-C base pairs exhibit greater thermal stability as compared to A-T base pairs. Thus, hybridization involving complementary nucleic acids of higher G-C content will be more stable at higher temperatures.

The ionic strength and incubation temperature under which a probe will be used should also be taken into account. It is known that hybridization will increase as the ionic strength of the reaction mixture increases, and that the thermal stability of molecular hybrids will increase with increasing ionic strength. On the other hand, chemical reagents such as formamide, urea, DMSO and alcohols, which disrupt hydrogen bonds, increase the stringency of hybridization. Destabilization of hydrogen bonds by such reagents can greatly reduce the Tm. In general, optimal hybridization for synthetic oligonucleotide probes of about 10-50 bases in length occurs approximately 5° C below the melting temperature for a given duplex. Incubation at temperatures below the optimum may allow mismatched base sequences to hybridize and can therefore result in reduced specificity.

A probe molecule may be used for hybridizing to a sample suspected of possessing a Scarface 3 or Scarface 3 related nucleotide sequence. The hybridization reaction is carried out under suitable conditions of stringency.

Alternatively, such DNA molecules may be used in a number of techniques including their use as: (1) diagnostic tools to detect polymorphisms in DNA samples from a human or other mammal; (2) means for detecting and isolating homologs of Scarface 3 and related polypeptides from a DNA library potentially containing such sequences; (3) primers for hybridizing to related sequences for the purpose of amplifying those sequences; and (4) primers for altering the native Scarface 3 DNA sequences; as well as other techniques which rely on the similarity of the DNA sequences to those of the Scarface 3 DNA segments herein disclosed.

Once synthesized, oligonucleotide probes may be labeled by any of several well known methods. See e.g. Maniatis *et. al.,* Molecular Cloning (2d ed. 1989). Useful labels include radioisotopes, as well as non-radioactive reporting groups. Isotopic labels include H³, S³⁵, P³², I¹²⁵, Cobalt, and C¹⁸. Most methods of isotopic labeling involve the use of enzymes and include methods such as nick-translation, end-labeling, second strand synthesis, and reverse transcription. When using radio-labeled probes, hybridization can be detected by autoradiography, scintillation counting, or gamma counting. The detection method selected will depend upon the hybridization conditions and the particular radio isotope used for labeling.

Non-isotopic materials can also be used for labeling, and may be introduced internally into the sequence or at the end of the sequence. Modified nucleotides may be incorporated enzymatically or chemically, and chemical modifications of the probe may be performed during or after synthesis of the probe, for example, by the use of non-nucleotide linker groups. Non-isotopic labels include fluorescent molecules, ehemiluminescent molecules, enzymes, cofactors, enzyme substrates, haptens or other ligands. In a preferred embodiment of the invention, the length of an oligonucleotide probe is greater than or equal to about 18 nucleotides and less than or equal to about 50 nucleotides. Labeling of an oligonucleotide of the present invention may be performed enzymatically using [³²P]-labeled ATP and the enzyme T4 polynucleotide kinase.

### Vectors

Another aspect of the present invention relates to recombinant DNA cloning vectors and expression vectors comprising the nucleic acids of the present invention. The preferred nucleic acid vectors are those which comprise DNA, for example, SEQ ID NO:1.

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene desired in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. The practitioner also understands that the amount of nucleic acid or protein to be produced dictates, in part, the selection of the expression system. Regarding promoter sequences, inducible promoters are preferred because they enable high level, regulatable expression of an operably-linked gene. The skilled artisan will recognize a number of suitable promoters that respond to a variety of inducers, for example, carbon source, metal ions, and heat. Other relevant considerations regarding an expression vector include whether to include sequences for directing the localization of a recombinant protein. For example, a sequence encoding a signal peptide preceding the coding region of a gene is useful for directing the extracellular export of a resulting polypeptide.

A useful vector for expressing Scarface 3 comprises the Scarface 3 coding sequence (e.g. SEQ ID NO:1) having fused at the 3' end of the coding region the so-called "Flag tag." (Miceli, et al., 1994; J. Immunol. Methods 167: 279-287, herein incorporated by reference). This produces a Scarface 3 flag tag protein (viz. DYKDDDDK fused to C-terminus of Scarface 3). The Scarface 3 coding region was PCR amplified with gene specific primers. The 3' primer contained the Flag tag sequence. The PCR product was cloned into the BamHI and EcoRI sites of pcDNA3.1 and pLZRS/IB. Clones were obtained in E.coli and sequenced to confirm the Scarface 3 coding region. pLZRS/IB (the IB refers to IRES/Blasticidin) was constructed by cloning an IRES (internal ribosome entry sequence) and blasticidin resistance gene from the pBMNZIB vector (purchased from G. Nolan) into the pLZRS vector (purchased from G. Nolan, Stanford). The Scarface 3 flag PCR product was then cloned into the BamHI and EcoRI sites just upstream of the IRES sequence of pLZRS/IB. The IB retrovirus was generated with the pLZRS/IB retroviral DNA transfected into the Phoenix packaging cell line (purchased from G. Nolan, Stanford).

The present invention also provides a method for constructing a recombinant host cell capable of expressing the proteins of the invention, said method comprising transforming or otherwise introducing into a host cell a recombinant DNA vector that comprises an isolated DNA sequence that encodes a protein of this invention. A suitable host cell is any eucaryotic cell that can accomodate high level expression of an exogenously introduced gene or protein, and that will incorporate said protein into its membrane structure. Vectors for expression are those which comprise an Scarface 3 gene or fragment thereof, e.g. SEQ ID NO:1. Transformed host cells may be cultured under conditions well known to skilled artisans such that a protein of the present invention is expressed, thereby producing a recombinant Scarface 3 protein in the recombinant host cell.

For the purpose of identifying compounds having utility as modifiers of angiogenesis, it would be desirable to identify compounds that bind an Scarface 3 protein of the present invention, and/or modify its activity, or the ability to bind its receptor. A method for determining agents that bind the Scarface 3 protein, comprises contacting the Scarface 3 protein with a test compound and monitoring binding by any suitable means.

The instant invention provides a screening system for discovering compounds that bind the Scarface 3 protein, said screening system comprising the steps of:
a) preparing a Scarface 3 protein;
b) exposing said Scarface 3 protein to a test compound;
c) quantifying the binding of said compound to Scarface 3 protein by any suitable means, for example, by monitoring a change in the myelosuppressive activity of Scarface 3.

Utilization of the screening system described above provides a means to identify compounds that may alter the biological function of Scarface 3 proteins. This screening method may be adapted to large-scale, automated procedures such as a PANDEX® (Baxter-Dade Diagnostics) system, allowing for efficient high-volume screening for potential therapeutic agents.

In one embodiment, Scarface 3 is prepared as described herein, preferably using recombinant DNA technology. A test compound is introduced into a reaction vessel containing an Scarface 3 protein or fragment thereof. Binding of Scarface 3 by a test compound is determined by any suitable means. For example, in one method radioactively-labeled or chemically-labeled test compound may be used. Binding of the protein by the test compound is assessed, for example, by quantifying bound label versus unbound label using any suitable method.

Binding of a test compound may also be carried out by a method disclosed in U.S. Patent 5,585,277, which hereby is incorporated by reference. In this method, binding of a test compound to a protein is assessed by monitoring the ratio of folded protein to unfolded protein, for example, by monitoring sensitivity of said protein to a protease, or the binding of said protein by a specific antibody against the folded state of the protein.

The foregoing screening methods are useful for identifying agonists and/or antagonists of a Scarface 3 protein as a lead to a pharmaceutical compound useful, for example, in treating cancer. For example, a compound that binds Scarface 3, or related fragment thereof, is identified by combining a test compound with Scarface 3 under conditions that cause the protein to exist in a ratio of folded to unfolded states. If a test compound binds the folded state of the protein, the relative amount of folded protein will be higher than in the case of a test compound that does not bind the protein. The ratio of protein in the folded versus unfolded state is easily determinable by, for example, susceptibility to digestion by a protease, or binding to a specific antibody, or binding to chaperonin protein, or binding to any suitable surface.

### Therapeutic Applications

In one embodiment, the present invention relates to therapeutic applications for Scarface 3 and analogs thereof. For example, Scarface 3 can be administered to enhance wound healing such as in post-surgical procedures. In another embodiment, Scarface 3 can be used in treating cancer by inhibiting solid tumor growth and metastasis, comprising the administration of an therapeutically effective amount of Scarface 3.

For therapeutic use in wound healing or other use in which enhanced angiogenesis is advantageous, an effective amount of Scarface 3 protein is administered to an organism in need thereof in a dose between about 1 and 1000 ug/kg body weight. In practicing the methods contemplated, Scarface 3 can be administered in a single daily dose or in multiple doses per day. The amount per administration will be determined by the physician and depend on such factors as the nature and severity of the disease, and the age and general health of the patient.

In another embodiment, the present invention relates to methods for treating leukemia comprising the administration of an therapeutically effective amount of Scarface 3 to a patient in need thereof.

The Scarface 3 protein can be used clinically to suppress the growth of hematopoietic progenitor cells and/or to prevent the expansion of such cells during chemotherapy or radiotherapy, as a means to protect the stem cell population from DNA damage during such therapeutic treatments. For example, the Scarface 3 protein can be administered by any suitable means, for example, intravenously, for any suitable time period, before chemotherapy or radiation therapy. For example, Scarface 3 can be administered one time, or alternatively multiple times over a 24 hour to 48 hour time period. Thereafter, chemotherapy or radiation therapy may ensue and Scarface 3 administration terminated. After chemotherapy or radiation therapy the patient can receive cytokines to stimulate the proliferation of the stem cell population.

The present invention also provides a pharmaceutical composition comprising as the active agent a Scarface 3 polypeptide or fragment, or analog, or a pharmaceutically acceptable non-toxic salt thereof, and a pharmaceutically acceptable solid or liquid carrier. For example, compounds comprising Scarface 3 can be admixed with conventional pharmaceutical carriers and excipients, and used in the form of tablets, capsules, elixirs, suspensions, syrups, wafers, and the like. The compositions comprising Scarface 3 will contain from about 0.1% to 90% by weight of the active compound, and more generally from about 10% to 30%. The compositions may contain common carriers and excipients such as corn starch or gelatin, lactose, sucrose, microcrystalline cellulose, kaolin, mannitol, dicalcium phosphate, sodium chloride, and alginic acid. The compounds can be formulated for oral or parenteral administration.

For intravenous (IV) use, the Scarface 3 protein is administered in commonly used intravenous fluid(s) and administered by infusion. Such fluids, for example, physiological saline, Ringer's solution or 5% dextrose solution can be used.

For intramuscular preparations, a sterile formulation, preferably a suitable soluble salt form of the Scarface 3 protein, for example SEQ ID NO:2, such as the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate.

Skilled artisans will recognize that IC₅₀ values are dependent on the selectivity of the compound tested. For example, a compound with an IC₅₀ which is less than 10 nM is generally considered an excellent candidate for drug therapy. However, a compound which has a lower affinity, but is selective for a particular target, may be an even better candidate. The skilled artisan will recognize that any information regarding the binding potential, inhibitory activity, or selectivity of a particular compound is useful toward the development of pharmaceutical products.

The following examples more fully describe the present invention. Those skilled in the art will recognize that the particular reagents, equipment, and procedures described are merely illustrative and are not intended to limit the present invention in any manner.

### EXAMPLE 1

### RT-PCR Amplification of Scarface 3 from mRNA

The Scarface 3 gene is isolated by reverse transcriptase PCR (RT-PCR) using conventional methods. Total RNA from a tissue that expresses the Scarface 3 gene, for example, kidney or liver tissue, is prepared using standard methods. First strand cDNA synthesis is achieved using a commercially available kit (Superscript™ System; Life Technologies) by PCR in conjunction with specific primers directed at any suitable region of SEQ ID NO:1.

Amplification is carried out by adding to the first strand cDNA (dried under vacuum): 8 µl of 10X synthesis buffer (200 mM Tris-HCl, pH 8.4; 500 mM KCl, 25 mM MgCl₂, 1 ug/ul BSA); 68 µl distilled water; 1 µl each of a 10 uM solution of each primer; and 1 µl Taq DNA polymerase (2 to 5 U/µl). The reaction is heated at 94° C for 5 min. to denature the RNA/cDNA hybrid. Then, 15 to 30 cycles of PCR amplification are performed using any suitable thermal cycle apparatus. The amplified sample may be analyzed by agarose gel electrophoresis to check for an appropriately-sized fragment.

### EXAMPLE 2

### Production of a Vector for Expressing Scarface 3 in a Host Cell

An expression vector suitable for expressing Scarface 3 or fragment thereof in a variety of procaryotic host cells, such as *E. coli* is easily made. The vector contains an origin of replication (Ori), an ampicillin resistance gene (Amp) useful for selecting cells which have incorporated the vector following a tranformation procedure, and further comprises the T7 promoter and T7 terminator sequences in operable linkage to a Scarface 3 coding region. Plasmid pET11A (obtained from Novogen, Madison WI) is a suitable parent plasmid. pET11A is linearized by restriction with endonucleases NdeI and BamHI. Linearized pET11A is ligated to a DNA fragment bearing NdeI and BamHI sticky ends and comprising the coding region of the Scarface 3 gene as disclosed by SEQ ID NO:1.

The Scarface 3 gene used in this construction may be modified at the 5' end (amino terminus of encoded protein) in order to simplify purification of the encoded protein product. For this purpose, an oligonucleotide encoding 8 histidine residues is inserted at or near the 5' end of SEQ ID NO:1, so as to maintain the correct reading frame. Placement of the histidine residues at the amino terminus of the encoded protein serves to enable the IMAC one-step protein purification procedure.

### EXAMPLE 3

### Recombinant Expression and Purification of Scarface 3 Protein

An expression vector that carries a gene encoding Scarface 3 or fragment thereof and which gene is operably-linked to an expression promoter is transformed into *E. coli* BL21 (DE3) *(hsdS gal* lcIts857 *ind*1*Sam*7*nin*5*lac*UV5-T7gene 1) using standard methods. Transformants, selected for resistance to ampicillin, are chosen at random and tested for the presence of the vector by agarose gel electrophoresis using quick plasmid preparations. Colonies which contain the vector are grown in L broth and the protein product encoded by the vector-borne gene is purified by immobilized metal ion affinity chromatography (IMAC), essentially as described in US Patent 4,569,794.

Briefly, the IMAC column is prepared as follows. A metal-free chelating resin (e.g. Sepharose 6B IDA, Pharmacia) is washed in distilled water to remove preservative substances and infused with a suitable metal ion [e.g. Ni(II), Co(II), or Cu(II)] by adding a 50mM metal chloride or metal sulfate aqueous solution until about 75% of the interstitial spaces of the resin are saturated with colored metal ion. The column is then ready to receive a crude cellular extract containing the recombinant protein product.

After removing unbound proteins and other materials by washing the column with any suitable buffer, pH 7.5, the bound protein is eluted in any suitable buffer at pH 4.3, or preferably with an imidizole-containing buffer at pH 7.5.

### EXAMPLE 4

### Tissue Distributuion of Scarface 3 mRNA

Scarface 3 mRNA is detected in a mammalian tissue by Northern analysis. Total RNA from different tissues or cultured cells is isolated by a standard guanidine chloride/phenol extraction method, and poly-A⁺ RNA is isolated using oligo(dT)-cellulose type 7 (Pharmacia). Electrophoresis of RNA samples is carried out in formaldehyde followed by capillary transfer to Zeta-Probe TM nylon membranes (Bio-Rad, Hercules, Calif.). SEQ ID NO:1 is used as a template for generating probes using a MultiPrime™ random priming kit (Amersham, Arlington Heights, Ill.). The efficiency of the labeling reaction is approximately 4 x 10¹⁰ cpm incorporated per µg of template. The hybridization buffer contains 0.5M sodium phosphate, 7% SDS (wt/vol), 1% BSA (wt/vol), and 1 mM EDTA. Prehybridization is carried out in hybridization buffer at 65° C for 2 h, and ³²P-labeled probe is added and incubation continued overnight. Filters are washed in Buffer A (40 mM sodium phosphate pH 7.2, 5% SDS [wt/vol], 0.5% BSA [wt/vol], and 1 mM EDTA) at 65° C for 1 h, then in Buffer B (40 mM sodium phosphate, pH 7.2, 1% SDS [wt/vol], and 1 mM EDTA) at 65° C for 20 min. Filters are then air-dried and exposed to Kodak X-OMAT AR film at -80° C with an intensifying screen. Scarface 3 transcript is detected in multiple tissues including kidney and liver.

### EXAMPLE 5

### Detecting Ligands that Bind Scarface 3 Using a Chaperonin Protein Assay

The wells of an ELISA plate are coated with chaperonin by incubation for several hours with a 4 ug/ml solution of the protein in Tris-buffered Saline (TBS: 10 mM Tris-HCl, pH7.5, 0.2M NaCl). Plates are washed 3 times with TBS containing 0.1% Tween-20 (TBST). Then, a mixture of Scarface 3 protein (sufficient amount to saturate about 50% of the binding sites on chaperonin) and test compound (10⁻⁹ to 10⁻⁵ M) in about 50 µl volume is added to each well of the plate for an incubation of about 60 minutes. Aliquots from each well are then transferred to the wells of fresh plates and incubation is continued for 60 minutes at room temperature, followed by 3 washes with TBST. Next, about 50 µl of an antibody specific for Scarface 3 in 5% nonfat dry milk is added to each well for 30 minutes at room temperature. After washing, about 50 µl of goat anti-rabbit IgG alkaline phosphatase conjugate at an appropriate dilution in TBST plus 5% nonfat dry milk is added to each will and incubated 30 minutes at room temperature. The plates are washed again with TBST, and 0.1 ml of 1 mg/ml p-nitrophenylphosphate in 0.1% diethanolamine is added. Color development (proportional to bound alkaline phosphatase antibody conjugate) is monitored with an ELISA plate reader. When binding by the test ligand has occurred, ELISA analysis reveals Scarface 3 in solution at higher concentrations than in the absence of test ligand.

### EXAMPLE 6

### Production of an Antibody to Scarface 3 Protein

Substantially pure Scarface 3 protein or fragment thereof is isolated from transfected or transformed cells using any of the well known methods in the art, or by a method specifically disclosed herein. Concentration of protein is adjusted, for example, by filtration through an Amicon filter device such that the level is about 1 to 5 ug/ml. Monoclonal or polyclonal antibody is prepared as follows.

Monoclonal antibody can be prepared from murine hybridomas according to the method of Kohler and Milstein *(Nature,* 256, 495, 1975), or a modified method thereof. Briefly, a mouse is repetitively inoculated with a few micrograms of the Scarface 3 protein (e.g. SEQ ID NO:2) or fragment thereof, or fusion peptide thereof, over a period of a few weeks. The mouse is then sacrificed, and antibody producing cells of the spleen are isolated. Spleen cells are fused with mouse myeloma cells using polyethylene glycol. Fused cells that produce antibody are identified by any suitable immunoassay, for example, ELISA, as described in E. Engvall, *Meth. Enzymol.,* 70, 419, 1980.

Polyclonal antiserum can be prepared by well known methods *(See e.g.* J. Vaitukaitis *et.al. Clin. Endocirnol. Metab.* 33, 988, 1971) that involve immunizing suitable animals with a Scarface 3 protein, fragments thereof, or fusion proteins thereof. Small doses (e.g. nanogram amounts) of antigen administered at multiple intradermal sites are the most reliable method.

### EXAMPLE 7

### Assay For Scarface 3 Activity

Scarface 3 activity is assayed by measuring tyrosine-specific phosphorylation, essentially as described in S. Davis *et al., Cell,* 87, 1161-1169, 1996; and T. Stitt *et. al. Cell,* 80, 661-670, 1995, both of which are herein incorporated by reference. Briefly, assays are done using late-confluency T-75 flasks of human umbilical vein endothelial cells (available from Clonetics, Inc. San Diego, CA). Cells are serum starved in high glucose DMDM for 2 - 4 hr. Conditioned medium, plus and minus varying amounts of Scarface 3 protein from about 10 ng/ml to about 10 ug/ml, is placed on cells for 5 - 10 min. at 37°C. Cells are then lysed in 1% NP-40 in PBS containing 1 mM PMSF, 0.14 U/ml aprotinin, 1 mM EDTA, and 1 mM sodium orthovanadate. The lysates are immunoprecipitated with a phosphotyrosine specific rabbit antiserum, electrophoresed, and immunoblotted (Western Blot) to nylon membranes. Following incubation of the blots with HRP-conjugated goat anti-rabbit antibodies ( Caltag, Inc.) bands are visualized by ECL.

### EXAMPLE 8

### Assay For Scarface 3 Antagonist

Assay reactions are set up essentially as described in Example 7, accept that a compound to be tested for Scarface 3 antagonist activity is included at the step of adding Scarface 3 to conditioned medium for 5 - 10 min. prior to cell lysis. Multiple assays can be set up in which a constant amount of Scarface 3 is incubated with varying amounts of test compound, for example from about 10 ng/ml to about 100 ug/ml.

### EXAMPLE 9

### Expression of Scarface 3 in EMT6 Breast Tumor Cells Produces Tumor Regression In Vivo

EMT6 tumor cells, cloned from mammary tumor KHJJ, are non-immunogenic cells that metastasize to the lung, and form progressive tumors in syngeneic Balb/c mice (Sara Rockwell, Lab. Anim. Sci. 27 (5): 831-851). EMT6 cells were grown in DMEM-F12 media + 10% FBS (Gibco), and transfected with either pcDNA 3.1 plasmid (Invitrogen, Inc.), or a recombinant plasmid consisting of a Scarface 3 Flag-tagged cDNA cloned into pcDNA 3.1, and Lipofectamine Plus reagent (Gibco), according to the manufacturer's protocol. Cells were selected 24 hours after transfection in neomycin (G418) (Gibco) at 0.5 mg/ml for 7 days.

The growth rates of transfected cells were assayed by tritiated thymidine incorporation. Approximately 5 x 10³ cells were seeded per well in triplicate of a 96 well tray for both transfected cell lines. At time points of 1, 2, and 3 days following seeding, the cells were pulsed with 1 uCi of tritiated thymidine for 4 hours and then frozen at-80° C. Cells were then harvested onto filters and counted for tritium incorporation into DNA. The results showed no significant difference between the growth rates of the two tumor cell lines.

Transfected cells were then grown exponentially to subconfluency and trypsinized. Trypsinized cells were washed twice with Hanks balanced saline solution (HBSS) (Gibco) and resuspended in HBSS at 2.5 million cells per ml. Approximately 5 x 10⁵ cells were injected within one hour subcutaneously into the right flank of either syngeneic Balb/c mice, or athymic nude mice (Harlan Sprague Dawley). The mice were ear-tagged for identification, housed in microisolator cages, and fed water and food *ad libitum.*

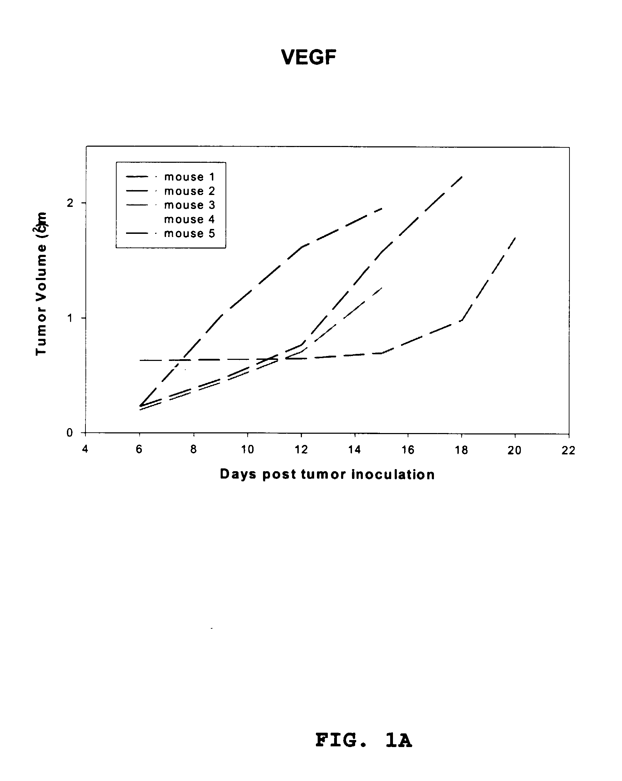

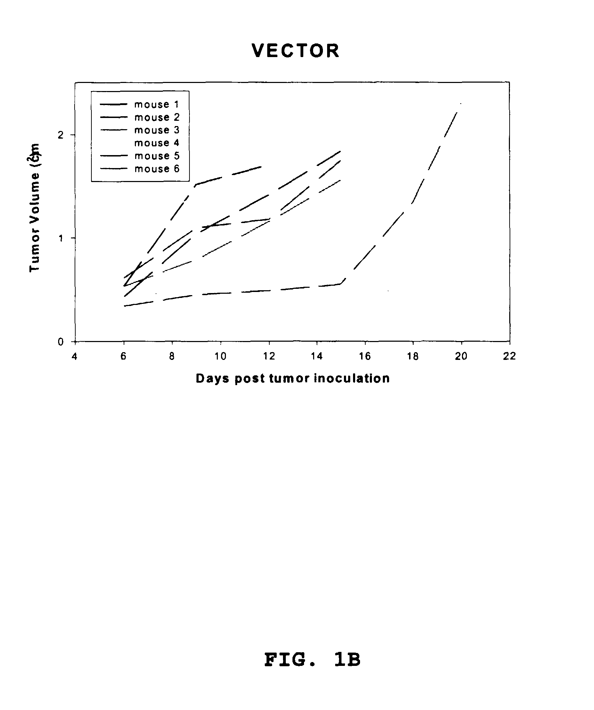

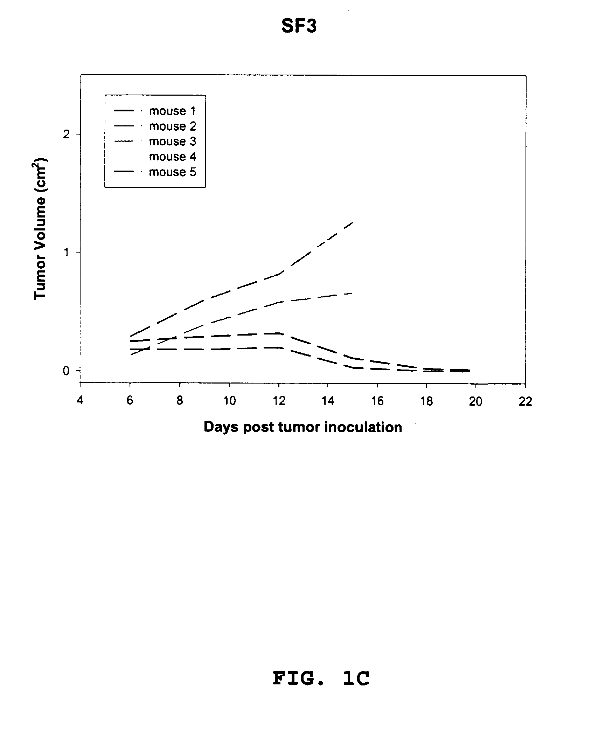

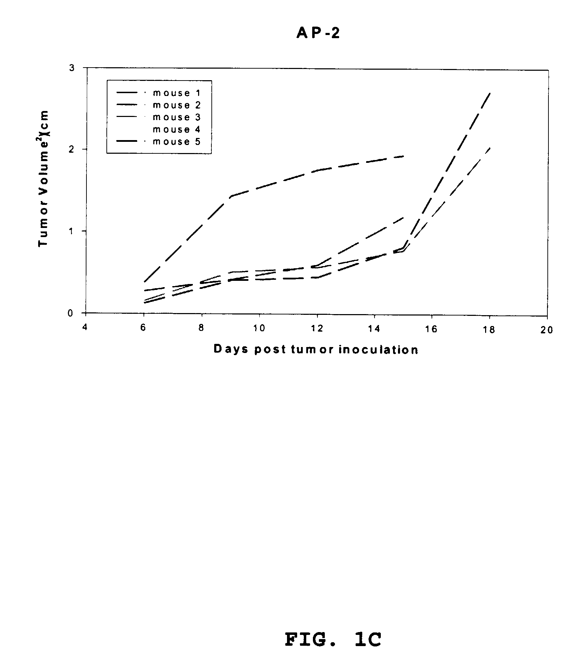

The results of these experiments are summarized in Table 2 and Figure 1. While no tumor regressed in athymic nude mice, which lack T-cells (data not shown), two of five Balb/C animals that received Scarface 3 transfected tumor cells exhibited regression of tumor size (Figure 1C). Tumors were measured with calipers in two dimensions in a blinded fashion every 2-3 days and tumor volume calculated by the formula: V= (1 x w²)/2.

### EXAMPLE 10

### Scarface 3 Induces Anti-tumor Immune Response In Vivo

Animals from Example 9 that exhibited tumor regression were injected in the left flank with 5 x 10⁵ wild-type EMT6 mammary carcinoma cells. As a control, several naive Balb/c mice were also injected with the same cells. After 12 days, the control animals exhibited large tumors, whereas the animals that rejected the tumor initially did not get wild-type tumors. This indicates that the animals showing regression developed an anti-tumor immune response to the poorly immunogenic wild type EMT6 cells. This response is most likely T-cell mediated, as athymic nude mice which lack T-cells did not reject the Scarface 3-expressing tumor cells.

### EXAMPLE 11

### Retroviral-mediated Murine Bone Marrow Cell Transduction

On Day -4, 5-Fluorouracil (5-FU, *Sigma, Cat# F6627, 5-Fluorouracil, 1g/bottle)* was resuspended in PBS to a stock concentration of 20/mg/ml, and diluted 1:4 in PBS (Final 5mg/ml) just before use. Each donor mouse was injected with 0.6 ml of diluted 5-FU intraperitoneally.

On Day 1 (after 4 days of 5FU treatment), mice were sacrificed and bone marrow (BM) cells isolated from each femur by standard techniques. Approxiamtely 1 x 10⁶ BM cells/ml in 8 ml of IMDM (containing 10% FBS) were deposited in a 10 cm Petri dish, and PBS added to make the final concentration 30%. Growth factors were added as follows:
rmSCF
Final Conc. = 100 ng/ml
Stock Conc. = 50 ug/ml
*(R&D systems, Cat#455-MC, recombinant mouse SCF, 50 ug/vial)*
rmIL-6
Final C = 100 U/ml
Stock C = 1 x 10⁵ U/ml
*(R&D systems, Cat#455-MC, recombinant mouse SCF, 50 ug/vial)*
Cells were incubated at 37°C for two days.

On Day 2, recombinant fibronectin (FN) fragment *(Panvera, Cat# Tad T100B, Retronectin, 2.5 mg/bottle)* was resuspended in PBS at a concentration of 2.5 mg/ml, and 2ml PBS added to each well of a 6 well non tissue culture-treated plate *(Falcon, Cat#1146).* Approximately 35ul of FN (~ 85ug) was added to each well and incubated at room temperature for at least 2 hours.

On Day 3, the FN/PBS solution was decanted from each well and each well blocked with 2ml 2% BSA *(Gibco BRL, Cat#15260-3=037, Bovine Albumin Fractin V Solution, 7.5%, 100 ml/bottle)* in PBS for 30 min at room temperature. Each well was washed twice with IMDM+ 10% FBS.

### First transduction:

Pre-stimulated BM cells were harvested and added to freshly thawed retroviral supernatant as follows: Approximately 4x10⁶ BM cells were added to each well in the 6 well plate, along with 3.2 ml viral supernatant (containing 10% FBS); 0.8 ml PBS (Final conc. PBS = 30%)
8 ul stock rm SCF, Final conc. 100ng/ml;
4 ul stock rm IL-6 Final C=100U/ml;
The plate was sealed and centrifuged at 180 RPM for 45 minutes at room temperature. BM cells were incubated in the viral supernatant at 37°C for 4 hours.

### Second transduction:

The viral supernatant and non-adherent BM cells were transferred from each well to a fresh tube, and fresh viral supernatant was added along with PBS and cytokines. The non-adherent BM cells were centrifuged and put back into the original wells. Cells were then incubated at 37°C overnight.

On Day 4, non-adherent BM cells were harvested by centrifugation, and each well washed twice with PBS. To each well was added 1 ml of cell dissociation buffer *(Gibco BRL, Cat# 13151-014, cell dissociate buffer, enzyme free, PBS-based, 100ml/bottle)* for 1-2 minutes at 37°C. Adherent BM cells were harvested by pipetting up and down several times followed by centrifugation. BM cells were counted and resuspended at 4x10⁶ /ml in PBS. Approximately 1-2x10⁶ BM cells (volume ≈ 0.5 ml) were injected into the tail vein of each irradiated recipient mouse.

### EXAMPLE 12

### CFU Assay for Scarface 3 and IB Retroviral Vector Transduced Murine Bone Marrow Cells

Murine bone marrow cells were collected from Balb/C mice after 3 days treatment with 5 fluorouracil. Harvested BM cells were pre-stimulated at 2 x 10⁶ cells/ml in IMDM plus 30% PBS, IL-6, and stem cell factor (SCF) for 2 days. Pre-stimulated BM cells were transduced twice with a Scarface 3F retrovirus (i.e. flag-tagged Scarface 3 sequence cloned in IB retrovirus), or a IB vector only control. Transduced cells were selected in blasticidin for 4 days. Blasticidin-resistant cells were harvested and used in a CFU assay.

Two different concentrations of the following cytokines were used in the CFU assay: GM-CSF (0 ng/ml; 50 ng/ml); Epo (4 U/ml; 1 U/ml); IL-3 (100 U/ml; 50 U/ml); SCF (100 ng/ml; 50 ng/ml). Approximately 1 x 10⁴ cells were plated in each dish. After seven days colonies comprising 3 different lineages were scored.

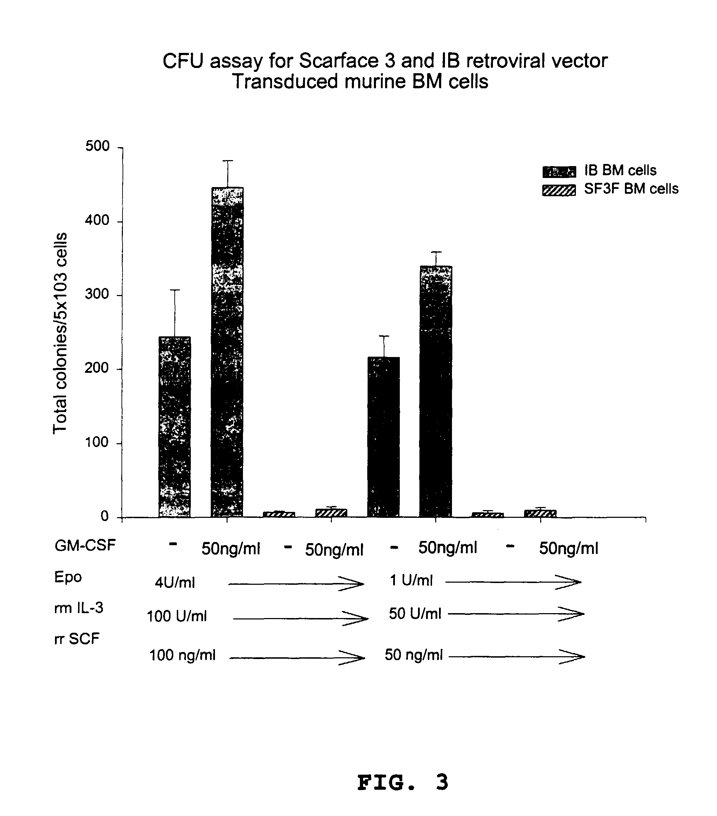

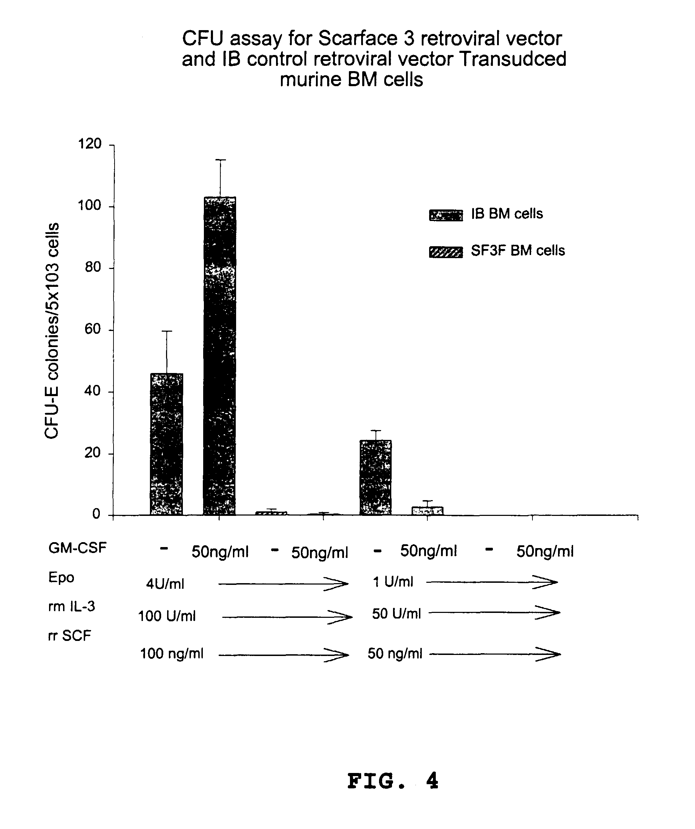

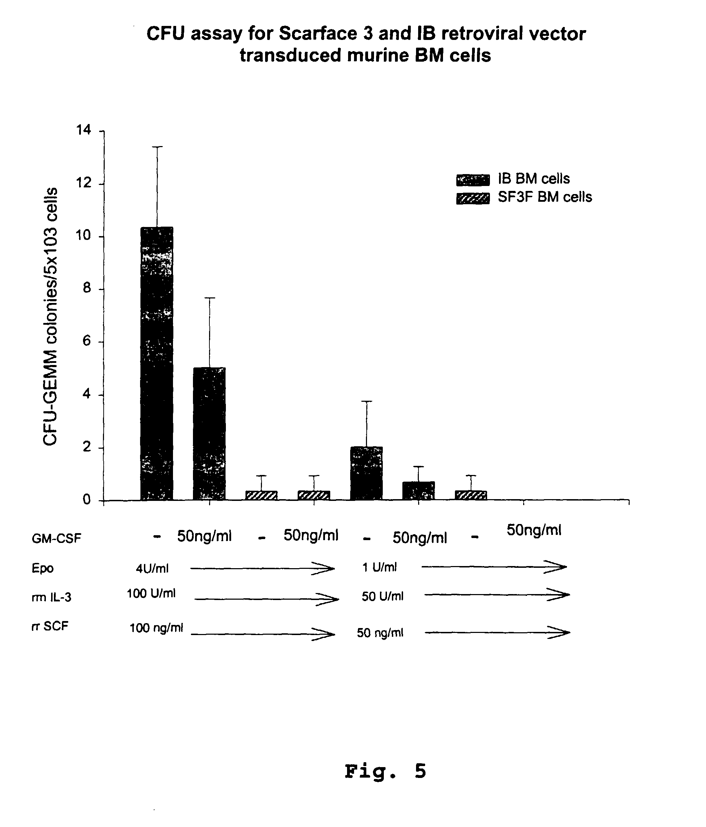

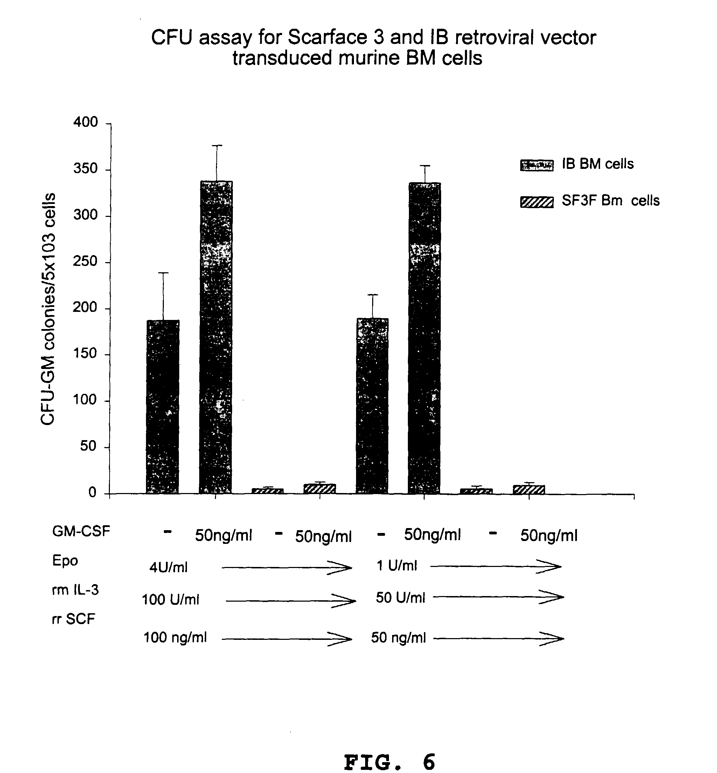

The results are summarized in Figures 3-6. All lineages of progenitors from Scarface 3 transduced BM cells were significantly reduced compared with the controls. For example, the total number of colonies recovered were suppressed some 50 to 100-fold (see Figure 3). At growth factor levels of 50 ng/ml GM-CSF, 4 U/ml Epo, 100 U/ml IL-3, and 100 ng/ml SCF, about 450 total colonies were observed in the control and less than 10 colonies in the Scarface 3 transduced cells. Specific cells types showed a similar suppressive effect. For example, Scarface 3 expression reduced about 100-fold the number of CFU-E colonies observed compared with the control (Figure 4). Similar observations were made respecting CFU-GEMM (Figure 5) and CFU-GM cells (Figure 6).

### EXAMPLE 13

### Scarface 3 Suppresses the Expansion of Progenitor Cells in Lethally Irradiated Mice Transplanted With Scarface 3 Transduced BM Cells

BM cells were harvested and transduced with an IB retrovirus carrying a Scarface 3F gene, or IB only as a negative control. Blasticidin resistant cells were selected and approximately 1 x 10⁶ resistant cells injected via the tail vein into lethally irradiated Balb/C mice as follows: Group A animals received 5 x 10⁵ cells transduced with IB; Group B animals received 2 x 10⁵ IB cells and 2 x 10⁵ cells transduced with the Scarface 3F expressing virus; Group C animals received 5 x 10⁵ cells transduced with Scarface 3F expressing virus.

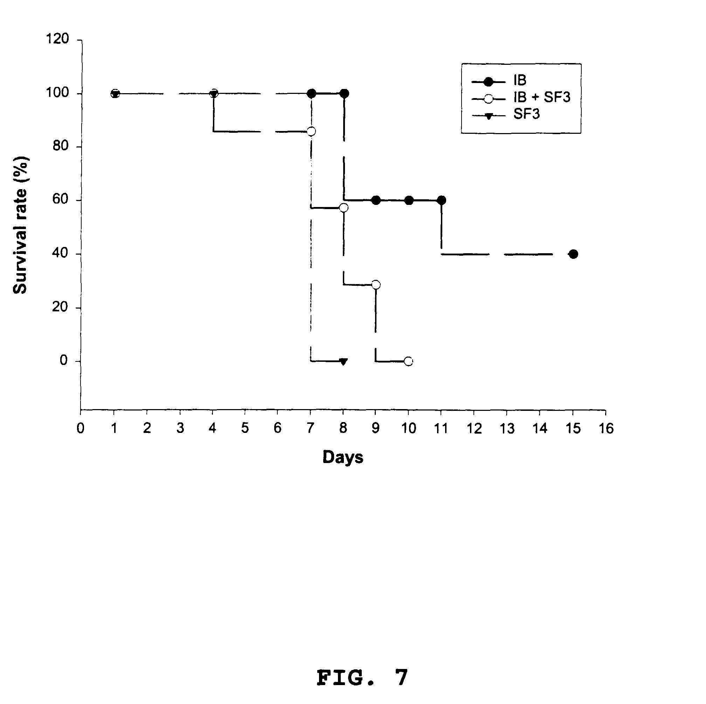

The results are summarized in Figure 7. Of 5 animals treated in Group A, 2 survived. On the other hand, 0/7 Group B animals survived, and 0/5 animals survived from Group C. That no mice survived the co-transplantation with Scarface 3 and control cells (Group B) suggests that Scarface 3 suppressed the expansion of progenitor cells from the control virus group.

In another experiment, 10 mice were transplanted with retroviral vector transduced BM cells and another 10 mice with Scarface 3 transduced BM cells. After ten days, all 10 animals of the vector only group survived; in contrast, 10/10 animals of the second group died.

### EXAMPLE 14

### Retroviral Transduction of TF-1 Cells With Scarface 3 Inhibits Proliferation

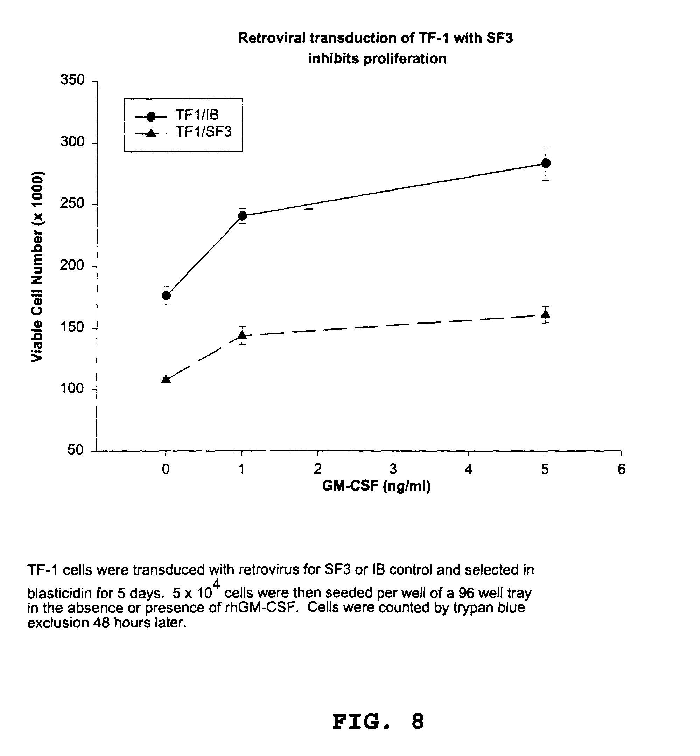

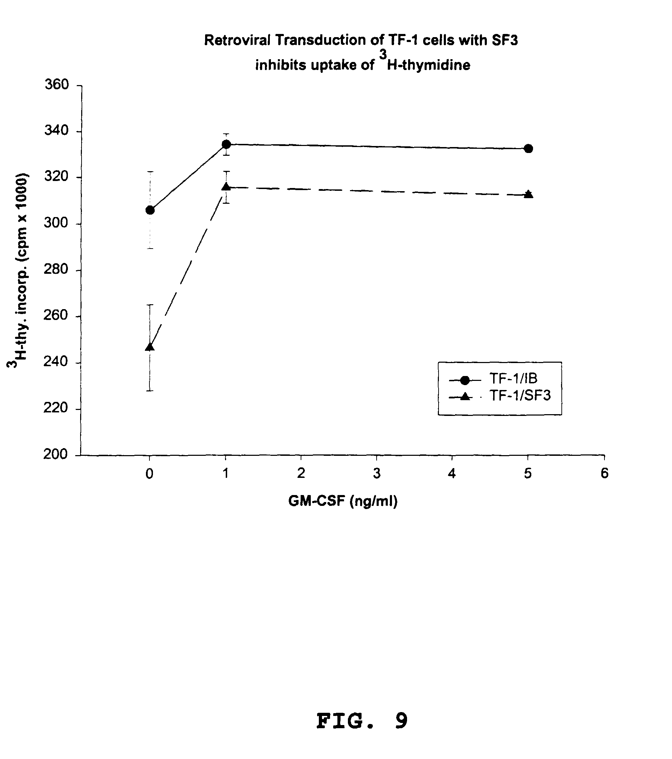

Human erythroleukemia cell line, TF-1, was transduced with IB retrovirus expressing a Scarface 3 cDNA, or with IB control vector lacking the Scarface 3 sequence. Approximately 5 x 10⁴ cells were seeded per well in a 96 well plate in the presence or absence of GM-CSF. Cells were counted by trypan blue exclusion 48 hours later (Figure 8). In a separate experiment, cells were pulsed 24 hours after plating with 1 uCi of ³H-thymidine and the amount of radiolabel incorporated in the cells determined 18 hours later by scintillation counting (Figure 9). The results showed that Scarface 3 expression inhibits the proliferation of an erythroleukemia cell line.

### EXAMPLE 15

### Identification of Critical Regions and Functional Analogs of Scarface 3

Critical regions of the Scarface 3 molecule can be identified by any number of suitable in vitro mutagenesis techniques, well known to the skilled artisan. One suitable method, known as Alanine-scanning mutagenesis (See Cunningham and Wells, Science, 244, 1081-1085, 1989), allows the introduction of individual alanine residues at each position of Scarface 3, as a test for the criticality of the replaced wild-type residue. The resulting mutated Scarface 3 molecules can be tested for retention of biological activity, for example, by assessing their ability to inhibit the proliferation of BM progenitor cells, as in Example 12.

The skilled artisan also recognizes that specific mutations can be constructed at defined sites in the wild-type Scarface 3 molecule to produce species of Scarface 3 analogs falling within the genus defined by SEQ ID NO:3. The resulting analogs can be tested without undue experimentation for function, for example, by the ability of the Scarface 3 analog(s) to inhibit the proliferation of BM progenitor cells. In this aspect, mutations defined according to SEQ ID NO:3 are targeted to the appropriate region of a wild-type Scarface 3 encoding nucleic acid, e.g. the cDNA defined by SEQ ID NO:1, using PCR oligonucleotide mutagenesis techniques, well known to the skilled artisan.

For example, the following single-substitution Scarface analogs are constructed by the above method(s), wherein the residue at position 247 is changed from Thr to Ala; the residue at position 249 is changed from Ile to Ala; the residue at position 272 is changed from Val to Ala; the residue at position 278 is changed from Ser to Ala; the residue at position 301 is changed from Asn to Ala; the residue at position 304 is changed from Tyr to Ala. The resulting Scarface 3 analogs are tested for biological activity as in Example 12.

### EXAMPLE 16

### Cytotoxic T lymphocye (CTL) activity from splenocytes of mice that rejected the EMT6/SF3 tumors.

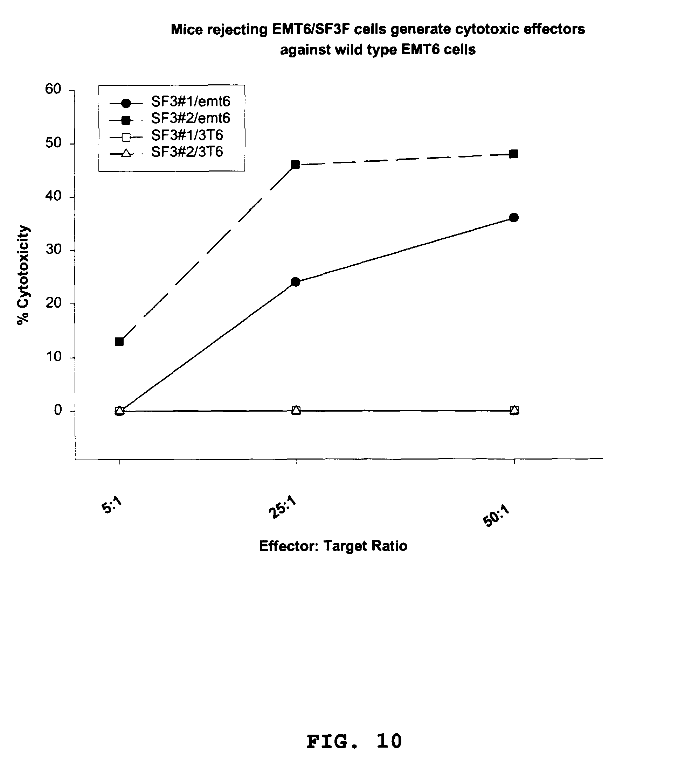

Spleens were removed from 2 mice that rejected the EMT6/Scarface 3 tumor (See Example 9) and stimulated in vitro for 5 days with irradiated EMT6 tumor cells. The CTLs (i.e. effector cells) from this culture were used in a CTL assay with EMT6 tumor cells as targets, or 3T6 cells as irrelevant target cells. The CTLs were incubated with the target cells (5 x 10³/well) at different ratios for 4 hours at 37° C in a U bottom 96 well plate, and then the amount of lactate dehydrogenase released into the supernatant was measured using a Cytotox 96 Kit (Promega Biotech). The percent cytotoxicity was calculated according to the manufacturer's instructions. The results showed that both mice had specific CTL activity against EMT6 cells but not the 3T6 cell line (See Figure 10).

**Table 2.**

| Tumor model study for Scarface 3 | | | | | |
|---|---|---|---|---|---|
| Animal | Body wt. | Liver wt. | Spleen | Thymus | Observations |
| **VEGT** | | | | | |
| 1051(A) | 21.4(g) | 1.64(g) | 459(mg) | 22(mg) | Spleen, enlarged, moderate Liver, whole tissue, pale, moderate Liver, cyst, clear, single |
| 1052 | 22.5 | 1.72 | 522 | 30 | Heart, lesions, white, multiple,05, collected Spleen, enlarged, moderate Thymus, small, slight Liver, whole tissue, pale, slight |
| 1053 | 23.3 | 1.58 | 337 | 39 | Heart, lesions, white, 05, multiple Spleen, enlarged, moderate |

| **Vector** | | | | | |
|---|---|---|---|---|---|
| 2051(B) | 23.6 | 1.59 | 420 | 25 | Heart, lesions, white, multiple, 05, collected Thymus, small, slight |
| 2052 | 23.6 | 1.56 | 415 | 49 | Heart, lesions, white, 05, few Spleen, enlarged, slight |
| 2053 | 21.4 | 1.38 | 338 | 22 | Tumor, fliud filled Thymus, small, slight |

| **Scarface 3** | | | | | |
|---|---|---|---|---|---|
| 3051(C) | 21.4 | 1.33 | 398 | 32 | Spleen, enlarged, moderate |
| 3052 | 24.6 | 1.63 | 429 | 27 | Spleen, enlarged, slight Tumor, fluid filled |
| 3053 | 20.3 | 1.32 | 228 | 38 | Tumor, fluid filled |

| **Angiopoietin 2** | | | | | |
|---|---|---|---|---|---|
| 4051(D) | 22.7 | 1.58 | 496 | 25 | Spleen, enlarged, mark Tumor, fluid filled |
| 4052 | 20.8 | 1.39 | 275 | 27 | Normal tissues |
| 4053 | 23.6 | 1.57 | 406 | 34 | Normal tissues |

## Claims

1. A substantially pure protein having an amino acid sequence that is at least 70% identical to SEQ ID NO: 2, said protein exhibiting Scarface 3-like activity in vivo or in vitro.

2. A substantially pure protein having an amino acid sequence that is encoded by a nucleic acid sequence that hybridizes to SEQ ID NO:1 under high stringency conditions said protein having Scarface 3 activity.

3. An analog of Scarface 3 having a sequence that is SEQ ID NO:3.

4. A substantially pure Scarface 3 protein having an amino acid sequence identified herein as SEQ ID NO: 2.

5. An isolated nucleic acid compound encoding a protein of Claim 1, Claim 2, Claim 3, or claim 4.

6. An isolated nucleic acid compound that encodes a Scarface 3 protein wherein said nucleic acid is SEQ ID NO:1.

7. A vector comprising an isolated nucleic acid claim 5 or 6.

8. A host cell containing a vector of Claim 7.

9. A pharmaceutical formulation comprising as an active ingredient an Scarface 3 protein or peptide, associated with one or more pharmaceutically acceptable carriers, excipients, or diluents thereof.

10. Scarface 3, as claimed in any one of claims 1 to 5, or a pharmaceutically acceptable salt thereof, for use as a pharmaceutical.


### Annex to the description

SEQUENCE LISTING

<110> Heuer, Josef G.

Burgett, Stanley G.

Little, Rebecca A.

Rosteck Jr., Paul R.

Sankhavaram, Patanjali R.

<120> Angiopoietin Related Gene Sequence Scarface 3

<130> X-12261

<140>

<141>

<160> 3

<170> PatentIn Ver. 2.0

<210> 1

<211> 1380

<212> DNA

<213> Homo sapiens

<220>

<221> CDS

<222> (1)..(1380)

<400> 1

atg ttc aca att aag ctc ctt ctt ttt att gtt cct cta gtt att tcc 48

Met Phe Thr Ile Lys Leu Leu Leu Phe Ile Val Pro Leu Val Ile Ser

1               5                   10                  15

tcc aga att gat caa gac aat tca tca ttt gat tct cta tct cca gag 96
Ser Arg Ile Asp Gln Asp Asn Ser Ser Phe Asp Ser Leu Ser Pro Glu
            20                  25                  30

cca aaa tca aga ttt gct atg tta gac gat gta aaa att tta gcc aat 144
Pro Lys Ser Arg Phe Ala Met Leu Asp Asp Val Lys Ile Leu Ala Asn
        35                  40                  45

ggc ctc ctt cag ttg gga cat ggt ctt aaa gac ttt gtc cat aag acg 192
Gly Leu Leu Gln Leu Gly His Gly Leu Lys Asp Phe Val His Lys Thr
    50                  55                  60

aag ggc caa att aat gac ata ttt caa aaa ctc aac ata ttt gat cag 240
Lys Gly Gln Ile Asn Asp Ile Phe Gln Lys Leu Asn Ile Phe Asp Gln
65                  70                  75                  80

tct ttt tat gat cta tcg ctg caa acc agt gaa atc aaa gaa gaa gaa 288
Ser Phe Tyr Asp Leu Ser Leu Gln Thr Ser Glu Ile Lys Glu Glu Glu
                85                  90                  95

aag gaa ctg aga aga act aca tat aaa cta caa gtc aaa aat gaa gag 336
Lys Glu Leu Arg Arg Thr Thr Tyr Lys Leu Gln Val Lys Asn Glu Glu
            100                 105                 110

gta aag aat atg tca ctt gaa ctc aac tca aaa ctt gaa agc ctc cta 384
Val Lys Asn Met Ser Leu Glu Leu Asn Ser Lys Leu Glu Ser Leu Leu
        115                 120                 125

gaa gaa aaa att cta ctt caa caa aaa gtg aaa tat tta gaa gag caa 432
Glu Glu Lys Ile Leu Leu Gln Gln Lys Val Lys Tyr Leu Glu Glu Gln
    130                 135                 140

cta act aac tta att caa aat caa cct gaa act cca gaa cac cca gaa 480
Leu Thr Asn Leu Ile Gln Asn Gln Pro Glu Thr Pro Glu His Pro Glu
145                 150                 155                 160

gta act tca ctt aaa act ttt gta gaa aaa caa gat aat agc atc aaa 528
Val Thr Ser Leu Lys Thr Phe Val Glu Lys Gln Asp Asn Ser Ile Lys
                165                 170                 175

gac ctt ctc cag acc gtg gaa gac caa tat aaa caa tta aac caa cag 576
Asp Leu Leu Gln Thr Val Glu Asp Gln Tyr Lys Gln Leu Asn Gln Gln
            180                 185                 190

cat agt caa ata aaa gaa ata gaa aat cag ctc aga agg act agt att 624
His Ser Gln Ile Lys Glu Ile Glu Asn Gln Leu Arg Arg Thr Ser Ile
        195                 200                 205

caa gaa ccc aca gaa att tct cta tct tcc aag cca aga gca cca aga 672
Gln Glu Pro Thr Glu Ile Ser Leu Ser Ser Lys Pro Arg Ala Pro Arg
    210                 215                 220

act act ccc ttt ctt cag ttg aat gaa ata aga aat gta aaa cat gat 720
Thr Thr Pro Phe Leu Gln Leu Asn Glu Ile Arg Asn Val Lys His Asp
225                 230                 235                 240

ggc att cct gct gaa tgt acc acc att tat aac aga ggt gaa cat aca 768
Gly Ile Pro Ala Glu Cys Thr Thr Ile Tyr Asn Arg Gly Glu His Thr
                245                 250                 255

agt ggc atg tat gcc atc aga ccc agc aac tct caa gtt ttt cat gtc 816
Ser Gly Met Tyr Ala Ile Arg Pro Ser Asn Ser Gln Val Phe His Val
            260                 265                 270

tac tgt gat gtt ata tca ggt agt cca tgg aca tta att caa cat cga 864
Tyr Cys Asp Val Ile Ser Gly Ser Pro Trp Thr Leu Ile Gln His Arg
        275                 280                 285

ata gat gga tca caa aac ttc aat gaa acg tgg gag aac tac aaa tat 912
Ile Asp Gly Ser Gln Asn Phe Asn Glu Thr Trp Glu Asn Tyr Lys Tyr
    290                 295                 300

ggt ttt ggg agg ctt gat gga gaa ttt tgg ttg ggc cta gag aag ata 960
Gly Phe Gly Arg Leu Asp Gly Glu Phe Trp Leu Gly Leu Glu Lys Ile
305                 310                 315                 320

tac tcc ata gtg aag caa tct aat tat gtt tta cga att gag ttg gaa 1008
Tyr Ser Ile Val Lys Gln Ser Asn Tyr Val Leu Arg Ile Glu Leu Glu
                325                 330                 335

gac tgg aaa gac aac aaa cat tat att gaa tat tct ttt tac ttg gga 1056
Asp Trp Lys Asp Asn Lys His Tyr Ile Glu Tyr Ser Phe Tyr Leu Gly
            340                 345                 350

aat cac gaa acc aac tat acg cta cat cta gtt gcg att act ggc aat 1104
Asn His Glu Thr Asn Tyr Thr Leu His Leu Val Ala Ile Thr Gly Asn
        355                 360                 365

gtc ccc aat gca atc ccg gaa aac aaa gat ttg gtg ttt tct act tgg 1152
Val Pro Asn Ala Ile Pro Glu Asn Lys Asp Leu Val Phe Ser Thr Trp
    370                 375                 380

gat cac aaa gca aaa gga cac ttc aac tgt cca gag ggt tat tca gga 1200
Asp His Lys Ala Lys Gly His Phe Asn Cys Pro Glu Gly Tyr Ser Gly
385                 390                 395                 400

ggc tgg tgg tgg cat gat gag tgt gga gaa aac aac cta aat ggt aaa 1248
Gly Trp Trp Trp His Asp Glu Cys Gly Glu Asn Asn Leu Asn Gly Lys
                405                 410                 415

tat aac aaa cca aga gca aaa tct aag cca gag agg aga aga gga tta 1296
Tyr Asn Lys Pro Arg Ala Lys Ser Lys Pro Glu Arg Arg Arg Gly Leu
            420                 425                 430

tct tgg aag tct caa aat gga agg tta tac tct ata aaa tca acc aaa 1344
Ser Trp Lys Ser Gln Asn Gly Arg Leu Tyr Ser Ile Lys Ser Thr Lys
        435                 440                 445

atg ttg atc cat cca aca gat tca gaa agc ttt gaa 1380
Met Leu Ile His Pro Thr Asp Ser Glu Ser Phe Glu
    450                 455                 460

<210> 2

<211> 460

<212> PRT

<213> Homo sapiens

<400> 2

Met Phe Thr Ile Lys Leu Leu Leu Phe Ile Val Pro Leu Val Ile Ser

1               5                   10                  15

Ser Arg Ile Asp Gln Asp Asn Ser Ser Phe Asp Ser Leu Ser Pro Glu

            20                  25                  30

Pro Lys Ser Arg Phe Ala Met Leu Asp Asp Val Lys Ile Leu Ala Asn

        35                  40                  45

Gly Leu Leu Gln Leu Gly His Gly Leu Lys Asp Phe Val His Lys Thr

    50                  55                  60

Lys Gly Gln Ile Asn Asp Ile Phe Gln Lys Leu Asn Ile Phe Asp Gln

65                  70                  75                  80

Ser Phe Tyr Asp Leu Ser Leu Gln Thr Ser Glu Ile Lys Glu Glu Glu

                85                  90                  95

Lys Glu Leu Arg Arg Thr Thr Tyr Lys Leu Gln Val Lys Asn Glu Glu

            100                 105                 110

Val Lys Asn Met Ser Leu Glu Leu Asn Ser Lys Leu Glu Ser Leu Leu
        115                 120                 125

Glu Glu Lys Ile Leu Leu Gln Gln Lys Val Lys Tyr Leu Glu Glu Gln
    130                 135                 140

Leu Thr Asn Leu Ile Gln Asn Gln Pro Glu Thr Pro Glu His Pro Glu
145                 150                 155                 160

Val Thr Ser Leu Lys Thr Phe Val Glu Lys Gln Asp Asn Ser Ile Lys
                165                 170                 175

Asp Leu Leu Gln Thr Val Glu Asp Gln Tyr Lys Gln Leu Asn Gln Gln
            180                 185                 190

His Ser Gln Ile Lys Glu Ile Glu Asn Gln Leu Arg Arg Thr Ser Ile
        195                 200                 205

Gln Glu Pro Thr Glu Ile Ser Leu Ser Ser Lys Pro Arg Ala Pro Arg
    210                 215                 220

Thr Thr Pro Phe Leu Gln Leu Asn Glu Ile Arg Asn Val Lys His Asp
225                 230                 235                 240

Gly Ile Pro Ala Glu Cys Thr Thr Ile Tyr Asn Arg Gly Glu His Thr
                245                 250                 255

Ser Gly Met Tyr Ala Ile Arg Pro Ser Asn Ser Gln Val Phe His Val
            260                 265                 270
Tyr Cys Asp Val Ile Ser Gly Ser Pro Trp Thr Leu Ile Gln His Arg
        275                 280                 285
Ile Asp Gly Ser Gln Asn Phe Asn Glu Thr Trp Glu Asn Tyr Lys Tyr
    290                 295                 300
Gly Phe Gly Arg Leu Asp Gly Glu Phe Trp Leu Gly Leu Glu Lys Ile
305                 310                 315                 320
Tyr Ser Ile Val Lys Gln Ser Asn Tyr Val Leu Arg Ile Glu Leu Glu
                325                 330                 335
Asp Trp Lys Asp Asn Lys His Tyr Ile Glu Tyr Ser Phe Tyr Leu Gly
            340                 345                 350
Asn His Glu Thr Asn Tyr Thr Leu His Leu Val Ala Ile Thr Gly Asn
        355                 360                 365
Val Pro Asn Ala Ile Pro Glu Asn Lys Asp Leu Val Phe Ser Thr Trp
    370                 375                 380
Asp His Lys Ala Lys Gly His Phe Asn Cys Pro Glu Gly Tyr Ser Gly
385                 390                 395                 400
Gly Trp Trp Trp His Asp Glu Cys Gly Glu Asn Asn Leu Asn Gly Lys

                405                 410                 415

Tyr Asn Lys Pro Arg Ala Lys Ser Lys Pro Glu Arg Arg Arg Gly Leu

            420                 425                 430

Ser Trp Lys Ser Gln Asn Gly Arg Leu Tyr Ser Ile Lys Ser Thr Lys

        435                 440                 445

Met Leu Ile His Pro Thr Asp Ser Glu Ser Phe Glu

    450                 455                 460

<210> 3

<211> 460

<212> PRT

<213> Artificial Sequence

<220>

<223> Description of Artificial Sequence: Scarface 3

analog

<220>

<223> Xaa at position 243 is Pro, Phe, or Trp

<220>

<223> Xaa at position 244 is Ala, Lys or Arg

<220>

<223> Xaa at position 245 is Glu or Asp

<220>
<223> Xaa at position 247 is Thr, Ala, Gln, or Leu

<220>
<223> Xaa at position 248 is Thr, Asp, Glu, or Gln

<220>
<223> Xaa at position 249 is Ile, Leu, Ala, or Val

<220>
<223> Xaa at position 250 is Tyr, Phe, Leu, or Lys

<220>
<223> Xaa at position 251 is Asn, Gln, Lys, or Glu

<220>
<223> Xaa at position 252 is Arg, Ala, Val, Ser, or Asp

<220>
<223> Xaa at position 254 is Glu, His, or Phe

<220>
<223> Xaa at position 255 is His, Arg, Ser, ASn, Thr, or
      Asp

<220>

<223> Xaa at position 256 is Thr, Lys, Val or Gln

<220>

<223> Xaa at position 257 is Ser or Asn

<220>

<223> Xaa at position 258 is Gly or Ser

<220>

<223> Xaa at position 259 is Met, Ile, or Leu

<220>

<223> Xaa at position 260 is Tyr or Phe

<220>

<223> Xaa at position 261 is Ala, Thr, Leu, Met or Glu

<220>

<223> Xaa at position 262 is Ile, Val or Leu

<220>

<223> Xaa at position 263 is Arg, Lys, Thr, Tyr or Gln

<220>

<223> Xaa at position 264 is Pro, Phe, or Ile

<220>

<223> Xaa at position 265 is Ser, Gln, Glu or Asn

<220>

<223> Xaa at position 266 is Asn or Gly


<220>

<223> Xaa at position 267 is Ser, Thr, or Met


<220>

<223> Xaa at position 268 is Gln, Glu, Pro, Arg, or Gly


<220>

<223> Xaa at position 269 is Val, Pro, Leu, or Glu


<220>

<223> Xaa at position 270 is Phe, Met, Ile, or Lys


<220>

<223> Xaa at position 271 is His, Lys, Gly, or Leu


<220>

<223> Xaa at position 272 is Val, Leu, or Ala


<220>

<223> Xaa at position 273 is Tyr, Phe, Trp, or Asn


<220>

<223> Xaa at position 275 is Tyr, Phe, Trp, or Asn

<220>

<223> Xaa at position 276 is Val, Met, Asn, or Gln

<220>

<223> Xaa at position 277 is Ile, Thr, Ser, Arg, Glu, or
      Asp

<220>

<223> Xaa at position 278 is Ser, Leu, His, Ala, or Val

<220>

<223> Xaa at position 279 is Gly, Pro, or Asp

<220>

<223> Xaa at position 280 is Ser, or Gly

<220>

<223> Xaa at position 281 is Pro, or Gly

<220>

<223> Xaa at position 284 is Leu, Val, or Ile

<220>

<223> Xaa at position 287 is His, Arg, or Lys

<220>

<223> Xaa at position 289 is Ile, Glu, Leu, Thr, or His

<220>

<223> Xaa at position 293 is Gln, Val, or Leu

<220>

<223> Xaa at position 294 is Asn, or Asp

<220>

<223> Xaa at position 296 is Asn, Phe, or Gln

<220>

<223> Xaa at position 297 is Glu, or Arg

<220>

<223> Xaa at position 298 is Thr, Gly, Asn, or Pro

<220>

<223> Xaa at position 300 is Glu, or Lys

<220>

<223> Xaa at position 301 is Asn, Glu, Thr, or Ala

<220>

<223> Xaa at position 304 is Tyr, Met, Val, Gln, Lys, or
      Ala

<220>

<223> Xaa at position 308 is Arg, Asn, or Asp

<220>

<223> Xaa at position 309 is Leu, Pro, or Ile

<220>

<223> Xaa at position 310 is Asp, Ser, or His

<220>

<400> 3

Met Phe Thr Ile Lys Leu Leu Leu Phe Ile Val Pro Leu Val Ile Ser
1               5                   10                  15

Ser Arg Ile Asp Gln Asp Asn Ser Ser Phe Asp Ser Leu Ser Pro Glu
            20                  25                  30

Pro Lys Ser Arg Phe Ala Met Leu Asp Asp Val Lys Ile Leu Ala Asn
        35                  40                  45

Gly Leu Leu Gln Leu Gly His Gly Leu Lys Asp Phe Val His Lys Thr
    50                  55                  60

Lys Gly Gln Ile Asn Asp Ile Phe Gln Lys Leu Asn Ile Phe Asp Gln
65                  70                  75                  80

Ser Phe Tyr Asp Leu Ser Leu Gln Thr Ser Glu Ile Lys Glu Glu Glu
                85                  90                  95

Lys Glu Leu Arg Arg Thr Thr Tyr Lys Leu Gln Val Lys Asn Glu Glu

            100                 105                 110

Val Lys Asn Met Ser Leu Glu Leu Asn Ser Lys Leu Glu Ser Leu Leu
        115                 120                 125

Glu Glu Lys Ile Leu Leu Gln Gln Lys Val Lys Tyr Leu Glu Glu Gln
    130                 135                 140

Leu Thr Asn Leu Ile Gln Asn Gln Pro Glu Thr Pro Glu His Pro Glu
145                 150                 155                 160

Val Thr Ser Leu Lys Thr Phe Val Glu Lys Gln Asp Asn Ser Ile Lys
                165                 170                 175

Asp Leu Leu Gln Thr Val Glu Asp Gln Tyr Lys Gln Leu Asn Gln Gln
            180                 185                 190

His Ser Gln Ile Lys Glu Ile Glu Asn Gln Leu Arg Arg Thr Ser Ile
        195                 200                 205

Gln Glu Pro Thr Glu Ile Ser Leu Ser Ser Lys Pro Arg Ala Pro Arg
    210                 215                 220

Thr Thr Pro Phe Leu Gln Leu Asn Glu Ile Arg Asn Val Lys His Asp
225                 230                 235                 240

Gly Ile Xaa Xaa Xaa Cys Xaa Xaa Xaa Xaa Xaa Xaa Gly Xaa Xaa Xaa
                245                 250                 255

Xaa Xaa Xaa Xaa Xaa Xaa Xaa Xaa Xaa Xaa Xaa Xaa Xaa Xaa Xaa Xaa
            260                 265                 270

Xaa Cys Xaa Xaa Xaa Xaa Xaa Xaa Xaa Trp Thr Xaa Ile Gln Xaa Arg
        275                 280                 285

Xaa Asp Gly Ser Xaa Xaa Phe Xaa Xaa Xaa Trp Xaa Xaa Tyr Lys Xaa
    290                 295                 300

Gly Phe Gly Xaa Xaa Xaa Gly Glu Xaa Trp Leu Gly Xaa Glu Xaa Xaa
305                 310                 315                 320

Xaa Xaa Xaa Xaa Xaa Xaa Xaa Xaa Xaa Xaa Leu Xaa Xaa Xaa Xaa Xaa
                325                 330                 335

Asp Trp Xaa Xaa Xaa Xaa Xaa Xaa Xaa Xaa Xaa Xaa Xaa Xaa Xaa Xaa
            340                 345                 350

Xaa Xaa Xaa Xaa Xaa Tyr Xaa Xaa Xaa Leu Xaa Xaa Xaa Xaa Gly Xaa
        355                 360                 365

Xaa Xaa Xaa Xaa Xaa Xaa Xaa Xaa Xaa Xaa Xaa Xaa Phe Xaa Thr Xaa
    370                 375                 380

Asp Xaa Xaa Xaa Xaa Xaa Xaa Xaa Xaa Cys Xaa Xaa Xaa Xaa Xaa Gly
385                 390                 395                 400

Gly Trp Trp Trp Xaa Xaa Xaa Cys Xaa Xaa Xaa Asn Leu Asn Gly Xaa
                405                 410                 415
Xaa Xaa Xaa Xaa Xaa Xaa Xaa Xaa Xaa Pro Glu Arg Arg Arg Gly Leu
            420                 425                 430
Ser Trp Lys Ser Gln Asn Gly Arg Leu Tyr Ser Ile Lys Ser Thr Lys
        435                 440                 445
Met Leu Ile His Pro Thr Asp Ser Glu Ser Phe Glu
    450                 455                 460